# EUROPEAN PATENT APPLICATION

(11) **EP 3 628 324 A1**
(43) Date of publication of application: **01.04.2020**
(21) Application number: 19192065.1
(22) Date of filing: 04.11.2014
(51) Int. Cl.: A61K 38/17, A61K 47/60, A61P 27/02

(54) **METHOD OF TREATING CONDITIONS OF THE EYE WITH AN ANTI-VEGF DARPIN**

(30) Priority: 05.11.2013 US 201361900246 P; 24.06.2014 US 201462016620 P
(62) Divisional of application: 14802544.8
(71) Applicant: ALLERGAN, INC., Irvine, CA 92612 (US)
(72) Inventor: Hohman, Thomas, Lake Forest, CA California 92630 (US); Cheetham, Janet, Laguna Niguel, CA California 92607 (US); Whitcup, Scott, Laguna Hills, CA California 92653 (US); Lippa, Erik, Irvine, CA California 91603 (US)
(74) Representative: Hoffmann Eitle

(57) **Abstract**

Disclosed herein are methods for the treatment of a patient having an exudative age-related macular degeneration and other conditions of the retina by administering a binding protein comprising an ankyrin repeat domain, wherein the binding protein is first administered in 2 to 5 doses, with an interval of 25 to 35 days between each dose, and then is administered in additional doses with a longer interval between doses.

## Description

### CROSS-REFERENCE TO RELATED APPLICATIONS

This application claims the benefit of U.S. Provisional Application No. 62/016,620 filed on June 24, 2014, and U.S. Provisional Application No. 61/900,246 filed on November 5, 2013. The entire contents of both of these applications are incorporated herein by reference.

### BACKGROUND

The retina is a thin layer of neural tissue lining the inner surface of the back of the eye. The retina consists of various kinds of neurons, the most familiar of which are the photoreceptors responsible for vision: rods, which are more sensitive to light, and cones, which are more sensitive to color. The cones are highly concentrated in the macula, the small, central portion of the retina. It is this portion of the eye that is responsible for central, high-acuity vision.

Diseases of the retina, and the macula in particular, are the leading cause of vision impairment and blindness in people over 50 years of age in developed countries. These diseases include age-related macular degeneration, myopic macular degeneration, diabetic macular edema, diabetic retinopathy, branch retinal vein occlusion, and central retinal vein occlusion. Macular degeneration can occur in children, as well.

Some of these diseases can be treated with agents that inhibit a protein called vascular endothelial growth factor A (VEGF-A). VEGF-A stimulates the growth of blood vessels. In the exudative (or vascular) form of age-related macular degeneration, abnormally high levels of VEGF stimulate the growth of new blood vessels into the macula causing irreversible damage to photoreceptors; in addition, these newly formed blood vessels leak blood and proteins into the retina, causing a scar to form in the area that was previously occupied by photoreceptors. Inhibiting VEGF-A blocks the formation of these new vessels, and blocks the leakage of blood and proteins, thus preserving vision.

There are only a few drugs that are currently used to inhibit VEGF-A in the eye: bevacizumab, ranibizumab, aflibercept, and pegaptanib. Ranibizumab is an antibody fragment that inhibits VEGF-A; bevacizumab is a humanized monoclonal antibody that also inhibits VEGF-A, and is derived from the same parent antibody as ranibizumab. Aflibercept is a recombinant fusion protein comprising extracellular domains of human VEGF receptors 1 and 2 fused to the Fc portion of human IgG1. Pegaptanib is not as effective as ranibizumab, bevacizumab, or aflibercept and so is not as frequently used.

In effect, then, there are only two therapies that may be used to inhibit VEGF in the eye: bevacizumab/ranibizumab and aflibercept. There is therefore a need in the art for therapies capable of treating diseases of the retina that are more effective than, or that provide different benefits from, the few therapies that are available.

### SUMMARY OF THE INVENTION

The inventors have discovered that the methods of the invention may be used to treat exudative age-related macular degeneration and other conditions of the retina with surprising benefits to the patient. In one embodiment, the method comprises delivering frequent doses of a recombinant binding protein, followed by less frequent doses.

Accordingly, in one embodiment of the invention, one administers about 0.25 mg to about 4 mg of the binding protein to a patient, and then administers the same dose 25 to 35 days later; one can then optionally administer the binding protein up to three times more, with the same interval between doses. After these initial, frequent doses, one continues to administer the binding protein, giving at least one additional dose, but with an interval of 50 to 115 days between each additional dose. Treatment is continued this way, administering the binding protein at 50 to 115 day intervals, for as long as the patient requires it.

Here is an example:

| **AMOUNT GIVEN** | **WHEN GIVEN** |
|---|---|
| 2 mg | Initial dose |
| 2 mg | Four weeks after initial dose |
| 2 mg | Four weeks after previous dose |
| 2 mg | Twelve weeks after previous dose |
| 2 mg | Twelve weeks after previous dose, and so on. |

This method may be used to improve visual acuity in a patient having a disease of the retina, to reduce abnormal fluid in the retina, and to reduce abnormal retinal thickness.

These and other embodiments are described in greater detail below.

### BRIEF DESCRIPTION OF THE FIGURES

**Figure 1** shows best-corrected visual acuity ("BCVA") at baseline and at weeks 1, 4, 8, 12, 16, and 20 in patients having exudative age-related macular degeneration ("AMD") receiving 1 mg and 2 mg of a binding protein according to the invention (abicipar), at day 1 and at weeks 4 and 8, and in patients receiving 0.5 mg of ranibizumab at day 1 and at weeks 4, 8, 12 and 16. Figures 2-7 also show data from patients treated in this way.
**Figure 2** shows the proportion of patients with a 15 or more letter improvement in BCVA in the three patient groups described above. 2.0 mg abicipar is the left bar in the set of three bars shown for each week; 1.0 mg abicipar is the middle bar; and 0.5 mg ranibizumab is the right bar.
**Figure 3** shows the proportion of patients who achieved stable vision, assessed as the loss of BCVA of less than 15 letters. 2.0 mg abicipar is the left bar in the set of three bars shown for each week; 1.0 mg abicipar is the middle bar; and 0.5 mg ranibizumab is the right bar.
**Figure 4** shows mean central retina thickness in patients receiving 2.0 mg abicipar, 1.0 mg abicipar, and 0.5 mg ranibizumab.
**Figure 5** shows the proportion of patients in whom intraretinal fluid, intraretinal cysts, and subretinal fluid (all three compartments) have resolved after treatment. 2.0 mg abicipar is the left bar in the set of three bars shown for each week; 1.0 mg abicipar is the middle bar; and 0.5 mg ranibizumab is the right bar.
**Figure 6** shows an image of the retina of a Caucasian male, 88 years of age, having baseline vision (Snellen) of 20/320 +1, and treated with 2 mg abicipar at baseline (day 1) and at weeks 4 and 8. Images shown are at baseline (Fig. 6A) and every four weeks thereafter (Figs. 6B-6G).
**Figure 7** shows an image of the retina of a Hispanic female, 75 years of age, having baseline vision (Snellen) of 20/63 +1, and treated with 2 mg abicipar at baseline (day 1) and at weeks 4 and 8. Images shown are at baseline (Fig. 7A) and every four weeks thereafter (Figs. 7B-7G).

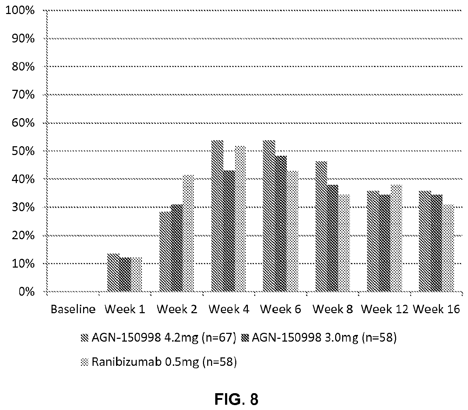

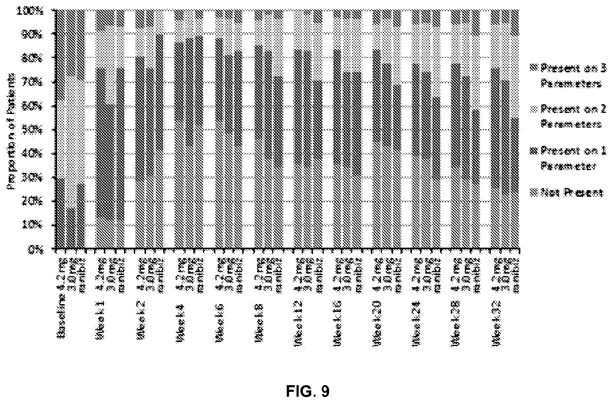

**Figures 8** **and** **9** show the proportion of patients in whom intraretinal fluid, intraretinal cysts, and subretinal fluid have resolved after treatment in a study of 175 patients having exudative AMD given 4.2 mg abicipar, 3.0 mg abicipar, and 0.5 mg ranibizumab in patients dosed on day 1 and then at week 16 or earlier if they met certain re-treatment criteria. **Figure 8** shows the proportion of patients in whom fluid in all three compartments has resolved; 4.2 mg abicipar is the left bar in the set of three bars shown for each week; 3.0 mg abicipar is the middle bar; and ranibizumab is the right bar. **Figure 9** shows the proportion of patients in whom fluid has resolved in one, two, all three, or none of these compartments.

### DETAILED DESCRIPTION OF THE INVENTION

### Recombinant Binding Proteins

The method of the invention administers a binding protein comprising a binding domain comprising a designed ankyrin repeat domain. Examples of such binding proteins and the designed ankyrin repeat domains they contain are described in U.S. Patent No. 7,417,130, U.S. Patent No. 8,110,653, and US Patent Application Publication No. 2011/0207668, the entire contents of all three of which are incorporated herein by reference.

The term "repeat proteins" refers to a protein comprising one or more repeat domains. In one embodiment, each of the repeat proteins comprises up to four repeat domains. In another embodiment, each of the repeat proteins comprises up to two repeat domains. In another embodiment, each of the repeat proteins comprises only one repeat domain. The repeat protein may also comprise additional non-repeat protein domains, polypeptide tags and/or polypeptide linkers.

The term "repeat domain" refers to a protein domain comprising two or more consecutive repeat units (modules) as structural units, wherein the structural units have the same fold, and stack tightly to create, for example, a superhelical structure having a joint hydrophobic core.

The term "designed repeat protein" and "designed repeat domain" refer to a repeat protein or repeat domain, respectively, obtained as the result of the procedure explained in U.S. Patent No. 7,417,130 and U.S. Patent No. 8,110,653. Designed repeat proteins and designed repeat domains are synthetic and not from nature. They are man-made proteins or domains, respectively, obtained by expression of correspondingly designed nucleic acids.

The mammalian VEGF family consists of five glycoproteins referred to as VEGF-A, VEGF-B, VEGF-C, VEGF-D (also known as FIGF) and placenta derived growth factor (PIGF, also known as PGF). VEGF-A has been shown to be an effective target for anti-angiogenic therapy (Ellis, L. M. and Hicklin, D. J., Nature Rev. Cancer 8, 579-591 , 2008). The VEGF-A ligands bind to and activate three structurally similar type III receptor tyrosine kinases, designated VEGFR- 1 (also known as FLT1), VEGFR-2 (also known as KDR) and VEGFR-3 (also known as FLT4). The VEGF ligands have distinctive binding specificities for each of these tyrosine kinase receptors, which contribute to their diversity of function. In response to ligand binding, the VEGFR tyrosine kinases activate a network of distinct downstream signaling pathways. VEGFR-1 and VEGFR-2 are primarily found on the vascular endothelium whereas VEGFR-3 is mostly found on the lymphatic endothelium. These receptors all have an extracellular domain, a single transmembrane region and a consensus tyrosine kinase sequence interrupted by a kinase-insert domain. More recently neuropilin (NRP-1), originally identified as a receptor for the semaphorin/collapsin family of neuronal guidance mediators, was shown to act as an isoform specific receptor for VEGF-A.

Various isoforms of VEGF-A are known that are generated by alternative splicing from eight exons within the VEGF-A gene. All isoforms contain exons 1-5 and the terminal exon, exon 8. Exons 6 and 7, which encode heparin-binding domains, can be included or excluded. This gives rise to a family of proteins termed according to their amino acid number: VEGF-A165, VEGF-A121, VEGF-A189, and so on. Exon 8, however, contains two 3' splice sites in the nucleotide sequences, which can be used by the cell to generate two families of isoforms with identical length, but differing C-terminal amino acid sequences (Varey, A.H.R. et al., British J. Cancer 98, 1366-1379, 2008). VEGF-Axxx ("xxx" denotes the amino acid number of the mature protein), the pro-angiogenic family of isoforms, is generated by use of the most proximal sequence in exon 8 (resulting in the inclusion of exon 8a). The more recently described anti-angiogenic VEGF-Axxxb isoforms are generated by the use of a distal splice site, 66 bp further along the gene from the proximal splice site. This results in splicing out of exon 8a and the production of mRNA sequences that encode the VEGF-Axxxb family. VEGF-A165 is the predominant pro-angiogenic isoform and is commonly overexpressed in a variety of human solid tumors. VEGF-A165b was the first of the exon 8b-encoded isoforms identified and was shown to have anti-angiogenic effects (Varey et al., loc. cit; Konopatskaya, O. et al., Molecular Vision 12, 626-632, 2006). It is an endogenous inhibitory form of VEGF-A, which decreases VEGF-A induced proliferation and migration of endothelial cells. Although it can bind to VEGFR-2, VEGF-A165b binding does not result in receptor phosphorylation or activation of the downstream signaling pathways.

The term "protein" refers to a polypeptide, wherein at least part of the polypeptide has, or is able to acquire a defined three-dimensional arrangement by forming secondary, tertiary, or quaternary structures within and/or between its polypeptide chain(s). If a protein comprises two or more polypeptides, the individual polypeptide chains may be linked non-covalently or covalently, e.g. by a disulfide bond between two polypeptides. A part of a protein, which individually has, or is able to acquire a defined three-dimensional arrangement by forming secondary or tertiary structures, is termed "protein domain." Such protein domains are well known to the practitioner skilled in the art.

The term "recombinant" as used in recombinant protein, recombinant protein domain and the like, means that the polypeptides are produced by the use of recombinant DNA technologies well known by the practitioner skilled in the relevant art. For example, a recombinant DNA molecule (e.g. produced by gene synthesis) encoding a polypeptide can be cloned into a bacterial expression plasmid (e.g. pQE30, Qiagen). When such a constructed recombinant expression plasmid is inserted into a bacteria (e.g. E. coli), this bacteria can produce the polypeptide encoded by this recombinant DNA. The correspondingly produced polypeptide is called a recombinant polypeptide.

The term "polypeptide tag" refers to an amino acid sequence attached to a polypeptide/protein, wherein the amino acid sequence is useful for the purification, detection, or targeting of the polypeptide/protein, or wherein the amino acid sequence improves the physicochemical behavior of the polypeptide/protein, or wherein the amino acid sequence possesses an effector function. The individual polypeptide tags, moieties and/or domains of a binding protein may be connected to each other directly or via polypeptide linkers. These polypeptide tags are all well known in the art and are fully available to the person skilled in the art. Examples of polypeptide tags are small polypeptide sequences, for example, His, myc, FLAG, or Strep-tags or moieties such as enzymes (for example enzymes like alkaline phosphatase), which allow the detection of the polypeptide/protein, or moieties which can be used for targeting (such as immunoglobulins or fragments thereof) and/or as effector molecules.

The term "polypeptide linker" refers to an amino acid sequence, which is able to link, for example, two protein domains, a polypeptide tag and a protein domain, a protein domain and a non-polypeptide moiety such as polyethylene glycol or two sequence tags. Such additional domains, tags, non-polypeptide moieties and linkers are known to the person skilled in the relevant art. A list of example is provided in U.S. Patent No. 7,417,130 and U.S. Patent No. 8,110,653. Examples of such linkers are glycine-serine-linkers and proline-threonine-linkers of variable lengths. In one embodiment, the linkers have a length of between 2 and 24 amino acids; in another embodiment, the linkers have a length of between 2 and 16 amino acids.

The term "polypeptide" relates to a molecule consisting of one or more chains of multiple, i.e. two or more, amino acids linked via peptide bonds. In one embodiment, a polypeptide consists of more than eight amino acids linked via peptide bonds.

The term "polymer moiety" refers to either a proteinaceous polymer moiety or a non-proteinaceous polymer moiety. In one embodiment, a "proteinaceous polymer moiety" is a polypeptide that does not form a stable tertiary structure while not forming more than 10% (or, not more than 5%, not more than 2%, not more than 1 %, and not more than any detectable amount, as determined by size exclusion chromatography (SEC)) of oligomers or aggregates when stored at a concentration of about 0.1 mM in PBS at RT for one month. Such proteinaceous polymer moieties run at an apparent molecular weight in SEC that is higher than their effective molecular weight when using globular proteins as molecular weight standards for the SEC. In one embodiment, the apparent molecular weight of the proteinaceous polymer moieties determined by SEC is 1.5x, 2x or 2.5x higher than their effective molecular weight calculated from their amino acid sequence. In one embodiment, the apparent molecular weights of the non-proteinaceous polymer moieties determined by SEC is 2x, 4x or 8x higher than their effective molecular weight calculated from their molecular composition. In one embodiment, more than 50%, 70% or even 90% of the amino acids of the proteinaceous polymer moiety do not form stable secondary structures at a concentration of about 0.1 mM in PBS at RT as determined by Circular Dichroism (CD) measurements. In one embodiment, the proteinaceous polymer shows a typical near UV CD-spectra of a random coil conformation. Such CD analyses are well known to the person skilled in the art. One can also use proteinaceous polymer moieties that comprise more than 50, 100, 200, 300, 400, 500, 600, 700 or 800 amino acids.

Examples of proteinaceous polymer moieties are XTEN® (a registered trademark of Amunix; WO 07/103515) polypeptides, or polypeptides comprising proline, alanine and serine residues as described in WO 08/155134. Such proteinaceous polymer moieties can be covalently attached to, for example, a binding domain of the invention by the generation of genetic fusion polypeptides using standard DNA cloning technologies, followed by their standard expression and purification. Examples of binding proteins comprising a repeat domain binding VEGF-Axxx and such a proteinaceous polymer moiety are shown in SEQ ID NO:1 and SEQ ID NO:4. The amino acid positions from 1 to 159 of SEQ ID NO:1 correspond to the repeat domain and the amino acid position 161 to 1 'O25 of SEQ ID NO:1 correspond to the proteinaceous polymer moiety. The amino acid positions from 1 to 126 of SEQ ID NO:4 correspond to the repeat domain and the amino acid positions 131 to 640 of SEQ ID NO:4 correspond to the proteinaceous polymer moiety.

A polymer moiety of the invention may vary widely in molecular weight (i.e. from about 1 kDa to about 150 kDa). In one embodiment, the polymer moiety has a molecular weight of at least 2 kDa, 5 kDa, 10 kDa, 20 kDa, 30 kDa, 50 kDa, 70 kDa or 100 kDa.

In one embodiment, the polymer moiety is connected by a polypeptide linker to a binding domain. Examples of such polypeptide linkers are the amino acids 1 to 8 of SEQ ID NO:8 and SEQ ID NO:9.

Examples of non-proteinaceous polymer moieties are hydroxyethyl starch (HES), polyethylene glycol (PEG), polypropylene glycol, or polyoxyalkylene. The term "PEGylated" means that a PEG moiety is covalently attached to, for example, a polypeptide of the invention. Examples of repeat proteins containing a polypeptide linker between the repeat domain and a C-terminal Cys residue useful for binding a non-proteinaceous polymer moiety are SEQ ID NO:2, 3, 5, 6 and 7. In some embodiments, PEG, if used, can be either in a linear or branched arrangement.

In a specific embodiment, a PEG moiety or any other non-proteinaceous polymer can, e.g., be coupled to a cysteine thiol via a maleimide linker with the cysteine being coupled via a peptide linker to the N- or C-terminus of a binding domain as described herein (e.g. SEQ ID NO:3).

The term "binding protein" refers to a protein comprising one or more binding domains and, in one embodiment, one or more polymer moieties as further explained below. In one embodiment, the binding protein comprises up to four binding domains. In one embodiment, the binding protein comprises up to two binding domains. In another embodiment, the binding protein has only one binding domain. Furthermore, any such binding protein may comprise additional protein domains that are not binding domains, multimerization moieties, polypeptide tags, polypeptide linkers and/or a single Cys residue. Examples of multimerization moieties are immunoglobulin heavy chain constant regions which pair to provide functional immunoglobulin Fc domains, and leucine zippers or polypeptides comprising a free thiol which forms an intermolecular disulfide bond between two such polypeptides. The single Cys residue may be used for conjugating other moieties to the polypeptide, for example, by using the maleimide chemistry well known to the person skilled in the art.

In one embodiment, the binding protein comprises up to four polymer moieties. In another embodiment, the binding protein comprises up to two polymer moieties. In another embodiment, the binding protein only has one polymer moiety.

In one embodiment, the binding protein has an apparent molecular weight of about 10 kDa, 15 kDa, 20 kDa, 25 kDa, 30 kDa, 35 kDa, 40 kDa, 45 kDa, 50 kDa, 55 kDa, 60 kDa, 65 kDa, 70 kDa, 75 kDa, 80 kDa, 85 kDa, 90 kDa, 95 kDa, 100 kDa, 200 kDa, 300 kDa, 400 kDa, 500 kDa, 600 kDa, 700 kDa, 800 kDa, 900 kDa, or 1,000 kDa when analyzed at a concentration of 0.1 mM in PBS at RT by SEC using globular proteins as molecular weight standards. In one embodiment, the binding protein has an apparent molecular weight of 34 kDa.

Those skilled in the art will appreciate the meaning of various terms of degree used herein. For example, as used herein in the context of referring to an amount, the term "about" represents those amounts close to and including the stated amount that still perform a desired function or achieve a desired result, e.g. "about 1 mg" includes 1 mg and those amounts reasonably close to 1 mg that still perform a desired function or achieve a desired result.

The term "binding domain" means a protein domain exhibiting the same "fold" (three-dimensional arrangement) as a protein scaffold and having a predetermined property, as defined below. Such a binding domain may be obtained by rational, or most commonly, combinatorial protein engineering techniques, skills which are known in the art (Skerra, 2000, loc. cit; Binz et al., 2005, loc. cit). For example, a binding domain having a predetermined property can be obtained by a method comprising the steps of (a) providing a diverse collection of protein domains exhibiting the same fold as a protein scaffold as defined further below; and (b) screening the diverse collection and/or selecting from the diverse collection to obtain at least one protein domain having the predetermined property. The diverse collection of protein domains may be provided by several methods in accordance with the screening and/or selection system being used, and may comprise the use of methods well known to the person skilled in the art, such as phage display or ribosome display.

The term "protein scaffold" means a protein with exposed surface areas in which amino acid insertions, substitutions or deletions are highly tolerable. Examples of protein scaffolds that can be used to generate binding domains of the present invention are antibodies or fragments thereof such as single-chain Fv or Fab fragments, protein A from Staphylococcus aureus, the bilin binding protein from Pieris brassicae or other lipocalins, ankyrin repeat proteins or other repeat proteins, and human fibronectin. Protein scaffolds are known to the person skilled in the art (Binz et al., 2005, loc. cit.; Binz et al., 2004, loc. cit.).

The term "predetermined property" refers to a property such as binding to a target, blocking of a target, activation of a target-mediated reaction, enzymatic activity, and related further properties. Depending on the type of desired property, one of ordinary skill will be able to identify format and necessary steps for performing screening and/or selection of a binding domain with the desired property. Preferably, the predetermined property is binding to a target.

In one embodiment, the binding domain of the invention does not comprise an immunoglobulin fold as present in antibodies and/or the fibronectin type III domain. An immunoglobulin fold is a common all-β protein fold that consists of a 2-layer sandwich of about 7 anti-parallel β-strands arranged in two β-sheets. Immunoglobulin folds are well known to the person skilled in the art. For example, such binding domains comprising an immunoglobulin fold are described in WO 07/080392 or WO 08/097497.

In another embodiment, the binding domain of the invention does not comprise an immunoglobulin-like domain as found in VEGFR-1 or VEGFR-2. Such binding domains are described in WO 00/075319.

In one embodiment, the binding domain comprises between 70 amino acids and 300 amino acids; in another embodiment, the binding comprises between 100 amino acids and 200 amino acids.

In one embodiment, the binding domain is devoid of a free Cys residue. A free Cys residue is not involved in the formation of a disulfide bond. In another embodiment, the binding domain is free of any Cys residue.

In one embodiment, the binding proteins of the invention may be expressed in eukaryotic or prokaryotic cells, such as bacterial cells, or by using a cell-free in vitro expression system.

The term "structural unit" refers to a locally ordered part of a polypeptide, formed by three-dimensional interactions between two or more segments of secondary structure that are near one another along the polypeptide chain. Such a structural unit exhibits a structural motif. The term "structural motif" refers to a three-dimensional arrangement of secondary structure elements present in at least one structural unit. Structural motifs are well known to the person skilled in the art. Structural units alone are not able to acquire a defined three-dimensional arrangement; however, their consecutive arrangement, for example as repeat modules in a repeat domain, leads to a mutual stabilization of neighboring units resulting in a superhelical structure.

The term "repeat unit" refers to amino acid sequences comprising repeat sequence motifs of one or more naturally occurring repeat proteins, wherein the "repeat units" are found in multiple copies, and which exhibit a defined folding topology common to all the motifs determining the fold of the protein. Examples of such repeat units are armadillo repeat units, leucine-rich repeat units, ankyrin repeat units, tetratricopeptide repeat units, HEAT repeat units, and leucine-rich variant repeat units. Naturally occurring proteins containing two or more such repeat units are referred to as "naturally occurring repeat proteins." The amino acid sequences of the individual repeat units of a repeat protein may have a significant number of mutations, substitutions, additions and/or deletions when compared to each other, while still substantially retaining the general pattern, or motif, of the repeat units.

The term "repeat sequence motif" refers to an amino acid sequence, which is deduced from one or more repeat units. In one embodiment, the repeat units are from repeat domains having binding specificity for the same target.

The term "folding topology" refers to the tertiary structure of the repeat units. The folding topology will be determined by stretches of amino acids forming at least parts of ohelices or β-sheets, or amino acid stretches forming linear polypeptides or loops, or any combination of a-helices, β-sheets and/or linear polypeptides/loops.

The term "consecutive" refers to an arrangement wherein the repeat units or repeat modules are arranged in tandem. In designed repeat proteins, there are at least 2 repeat units, but usually about 2 repeat units to 6 repeat units, but there may also be 6 or more repeat units or 20 or more repeat units. In most cases, repeat units will exhibit a high degree of sequence identity (same amino acid residues at corresponding positions) or sequence similarity (amino acid residues being different, but having similar physicochemical properties), and some of the amino acid residues might be key residues being strongly conserved in the different repeat units found in naturally occurring proteins. However, a high degree of sequence variability by amino acid insertions and/or deletions, and/or substitutions between the different repeat units found in naturally occurring proteins will be possible as long as the common folding topology is maintained.

Methods for directly determining the folding topology of repeat proteins by physico-chemical means such as X-ray crystallography, NMR or CD spectroscopy, are well known to the practitioner skilled in the art. Methods for identifying and determining repeat units or repeat sequence motifs or for identifying families of related proteins comprising such repeat units or motifs, such as homology searches (BLAST etc.), are well established in the field of bioinformatics, and are well known to the practitioner in the art. The step of refining an initial repeat sequence motif may comprise an iterative process.

The term "repeat modules" refers to the repeated amino acid sequences of the designed repeat domains, which are originally derived from the repeat units of naturally occurring repeat proteins. Each repeat module comprised in a repeat domain is derived from one or more repeat units of the family or subfamily of naturally occurring repeat proteins, e.g. the family of armadillo repeat proteins or ankyrin repeat proteins.

"Repeat modules" may comprise positions with amino acid residues present in all copies of corresponding repeat modules ("fixed positions") and positions with differing or "randomized" amino acid residues ("randomized positions").

The term "capping module" refers to a polypeptide fused to the N- or C-terminal repeat module of a repeat domain, wherein the capping module forms tight tertiary interactions with the repeat module thereby providing a cap that shields the hydrophobic core of the repeat module at the side not in contact with the consecutive repeat module from the solvent. The N- and/or C-terminal capping module may be, or may be derived from, a capping unit or other domain found in a naturally occurring repeat protein adjacent to a repeat unit. The term "capping unit" refers to a naturally occurring folded polypeptide, wherein the polypeptide defines a particular structural unit which is N- or C-terminally fused to a repeat unit, wherein the polypeptide forms tight tertiary interactions with the repeat unit thereby providing a cap that shields the hydrophobic core of the repeat unit at one side from the solvent. Such capping units may have sequence similarities to the repeat sequence motif. Capping modules and capping repeats are described in U.S. Patent No. 7,417,130 and U.S. Patent No. 8,110,653. For example, the N-terminal capping module of SEQ ID NO:2 is encoded by the amino acids from position 1 to 32. Also preferred is such an N-terminal capping module having a glycine or aspartate residue at position 5.

The term "target" refers to an individual molecule such as a nucleic acid molecule, a polypeptide or protein, a carbohydrate, or any other naturally occurring molecule, including any part of such individual molecule, or complexes of two or more of such molecules. The target may be a whole cell or a tissue sample, or it may be any non-natural molecule or moiety. Preferably, the target is a naturally occurring or non-natural polypeptide or a polypeptide containing chemical modifications, for example modified by natural or non-natural phosphorylation, acetylation, or methylation. In some embodiments, the target is VEGF-Axxx or VEGFR-2.

The term "consensus sequence" refers to an amino acid sequence, wherein the consensus sequence is obtained by structural and/or sequence aligning of multiple repeat units. Using two or more structural and/or sequence aligned repeat units, and allowing for gaps in the alignment, it is possible to determine the most frequent amino acid residue at each position. The consensus sequence is that sequence which comprises the amino acids which are most frequently represented at each position. In the event that two or more amino acids are represented above-average at a single position, the consensus sequence may include a subset of those amino acids. The two or more repeat units may be taken from the repeat units comprised in a single repeat protein, or from two or more different repeat proteins.

Consensus sequences and methods to determine them are well known to the person skilled in the art.

A "consensus amino acid residue" is the amino acid found at a certain position in a consensus sequence. If two or more, e.g. three, four or five, amino acid residues are found with a similar probability in the two or more repeat units, the consensus amino acid may be one of the most frequently found amino acids or a combination of the two or more amino acid residues.

In one embodiment, one may also use non-naturally occurring binding proteins or binding domains.

The term "non-naturally occurring" means synthetic or not from nature, for example, made by a person. The term "non-naturally occurring binding protein" or "non-naturally occurring binding domain" means that the binding protein or the binding domain is synthetic (i.e. produced by chemical synthesis from amino acids) or recombinant and not from nature. "Non-naturally occurring binding protein" or "non-naturally occurring binding domain" is a man-made protein or domain, respectively, obtained by expression of correspondingly designed nucleic acids. Preferably, the expression is done in eukaryotic or bacterial cells, or by using a cell-free in vitro expression system. Further, the term means that the sequence of the binding protein or the binding domain is not present as a non-artificial sequence entry in a sequence database, for example in GenBank, EMBL-Bank or Swiss-Prot. These databases and other similar sequence databases are well known to the person skilled in the art.

A binding domain can inhibit VEGF-Axxx binding to VEGFR-2 either by binding to VEGF-Axxx or by binding to VEGFR-2 in a way that the apparent dissociation constant (K_{d}) between VEGF-Axxx and VEGFR-2 is increased more than 10²-fold. In other embodiments, the dissociation constant is increased more than 10³-fold, more than 10⁴-fold, more than 10⁵-fold, or more than 10⁶-fold. In one embodiment, the K_{d} for the interaction of the binding domain to either VEGF-Axxx or VEGFR-2 is below 10⁷M, below 10⁸M, or below 10⁹M, below 10¹⁰M, or below 10¹¹M. Methods to determine dissociation constants of protein-protein interactions, such as surface plasmon resonance (SPR) based technologies, are well known to the person skilled in the art.

The binding domain binds VEGF-Axxx. In one embodiment, the binding domain binds human VEGF-A165. In other embodiments, it may bind other VEGF-A isoforms.

In one embodiment, the binding protein and/or binding domain do not lose their native three-dimensional structure upon incubation in PBS containing 100 mM dithiothreitol (DTT) for 1 or 10 hours at 37°C. "PBS," as used here, means a phosphate buffered water solution containing 137 mM NaCl, 10 mM phosphate and 2.7 mM KCI and having a pH of 7.4.

In one embodiment, the binding protein comprises a binding domain that inhibits VEGF-Axxx binding to VEGFR-2 and has the midpoint denaturation temperature and non-aggregating properties as defined above, wherein the binding protein inhibits sprouting of HUVEC spheroids with an IC₅₀ value below 100 nM.

The term "HUVEC" means human umbilical vein endothelial cells, which can be isolated from normal human umbilical vein and which are responsive to VEGF-A stimulation.

Assays to measure the sprouting of HUVEC spheroids are well known to the person skilled in the art.

An IC₅₀ value is the concentration of a substance, such as a binding protein or binding domain, which is required for 50% inhibition in vitro of an experimental determined parameter, such as the sprouting of HUVEC spheroids. IC₅₀ values can be readily determined by the person skilled in the art (Korff T. and Augustin H.G., J. Cell Biol. 143(5), 1341 -52, 1998).

In one embodiment, the binding protein and/or binding domain inhibit the sprouting of HUVEC spheroid with an IC₅₀ value below 10 nM, below 1 nM, below 0.1 nM, or below 0.05 nM.

In another embodiment, one can use a monomeric binding protein and/or binding domain that inhibit the sprouting of HUVEC spheroids with an IC₅₀ value lower than the corresponding IC₅₀ value of ranibizumab, bevacizumab, aflibercept, or pegaptanib.

In one embodiment, the K_{d} for the interaction of a binding domain to VEGF-B, VEGF-C, VEGF-D, PIGF or PDGF is above 1 nM, above 10 nM, above 10² nM, above 10³ nM, or above 10⁴ nM.

In one embodiment, VEGF-Axxx is either dog VEGF-A 164 or simian VEGF-A 165 or human VEGF-A165, and VEGF-Axxxb is either dog VEGF-A 164b or simian VEGF-A165b or human VEGF-A165b.

Another embodiment is a recombinant binding protein comprising a binding domain, wherein the binding domain inhibits VEGF-Axxx binding to VEGFR-2 and wherein the binding domain is a repeat domain or a designed repeat domain. Such a repeat domain may comprise one, two, three or more internal repeat modules that will participate in binding to VEGF-Axxx. In one embodiment, such a repeat domain comprises an N-terminal capping module, two to four internal repeat modules, and a C-terminal capping module. In one embodiment, the binding domain is an ankyrin repeat domain or designed ankyrin repeat domain.

In one embodiment the binding protein comprises a binding domain as described herein, conjugated to a polyethylene glycol (PEG) moiety. In one embodiment the PEG moiety is coupled to a single Cys residue of the binding domain. The Cys residue can be genetically introduced at the C-terminal end of the binding domain. The PEG moiety can then be coupled by chemical means, for example, by using maleimide chemistry well known to the person skilled in the art.

Another embodiment is a recombinant binding protein as defined above comprising at least one repeat domain with binding specificity for VEGF-Axxx, wherein the repeat domain competes for binding to VEGF-Axxx with a repeat domain selected from the group of the repeat domains of SEQ ID NO:1 to 7. In one embodiment, the repeat domain competes for binding to VEGF-Axxx with the repeat domain of SEQ ID NO:1 or 3. In another embodiment, the repeat domain competes for binding to VEGF-Axxx with the repeat domain of SEQ ID NO:3.

The term "compete for binding" means the inability of two different binding domains of the invention to bind simultaneously to the same target, while both are able to bind the same target individually. Thus, such two binding domains compete for binding to the target. Methods, such as competition ELISA or competition SPR measurements (e.g. by using the Proteon instrument from BioRad), to determine if two binding domains compete for binding to a target are well known to the practitioner in the art.

A recombinant binding protein that competes for binding to VEGF-Axxx with a selected repeat protein can be identified by methods well know to the person skilled in the art, such as a competition Enzyme-Linked Immunosorbent Assay (ELISA).

Another embodiment is a recombinant binding protein comprising a repeat domain with binding specificity for VEGF-Axxx selected from the group consisting of the repeat domains of SEQ ID NO:1 to 7. In one embodiment, the repeat domain is selected from the repeat domains of SEQ ID NO:2 or 3. In another embodiment, the repeat domain is the repeat domain of SEQ ID NO:3.

In one embodiment, the ankyrin repeat domain comprises a repeat module with the ankyrin repeat sequence motif
1D23G4TPLHLAA56GHLEIVEVLLK7GADVNA (SEQ ID NO: 10) wherein 1, 2, 3, 4, 5, 6, and 7, represent, independently of each other, an amino acid residue selected from the group A, D, E, F, H, I, K, L, M, N, Q, R, S, T, V, W and Y.

In another embodiment, the ankyrin repeat domain comprises a repeat module with the ankyrin repeat sequence motif
1D23GWTPLHLAA45GHLEIVEVLLK6GADVNA (SEQ ID NO: 11)
wherein 1 represents an amino acid residue selected from the group consisting of F, T, N, R, V, A, I, K, Q, S and Y; in one embodiment, 1 is F, T, N, R or V; and in another embodiment, 1 is F or T; 2 represents an amino acid residue selected from the group consisting of W, Y, H and F; in another embodiment, 2 is W, Y or H; 3 represents an amino acid residue selected from the group consisting of M, I, F and V; in another embodiment, 3 is M or I; 4 represents an amino acid residue selected from the group consisting of H, A, K, G, L, M, N, T, V, W and Y; in another embodiment, 4 is H, A or K; 5 represents an amino acid residue selected from the group consisting of E, Y, F, V, H, I, L, N and R; in another embodiment, 5 is E, Y, F, V or H; in another embodiment, 5 is E, Y, F or V; and 6 represents an amino acid residue selected from the group consisting of A, N, Y, H and R.

In another embodiment, the ankyrin repeat domain comprises a repeat module with the ankyrin repeat sequence motif
1D23G4TPLHLAA56GHLEIVEVLLK7GADVN8 (SEQ ID NO: 12)
wherein 1 represents an amino acid residue selected from the group consisting of T, E, A, D, F, K, N, Q, R, S, W and Y; in another embodiment, 1 is T or E; 2 represents an amino acid residue selected from the group consisting of V, F, Y, A, H, I, K, R, T and W; in another embodiment, 2 is V, F or Y; 3 represents an amino acid residue selected from the group consisting of S, A, N, F and M; in another embodiment, 3 is S, A or N; in another embodiment, 3 is S or A; 4 represents an amino acid residue selected from the group consisting of Y, F, S and W; 5 represents an amino acid residue selected from the group consisting of A, S, L and Y; in another embodiment, 5 is A or S; 6 represents an amino acid residue selected from the group consisting of D, N, M, A, I, K and Y; in another embodiment, 6 is D, N or M; in another embodiment, 6 is D or N; 7 represents an amino acid residue selected from the group consisting of A, Y, H, N and D; and 8 represents the amino acid residue T or A.

In another embodiment, the ankyrin repeat domain comprises a repeat module with the ankyrin repeat sequence motif
1D23GWTPLHL4ADLG5LEIVEVLLK6GADVN7 (SEQ ID NO: 13)
wherein 1 represents an amino acid residue selected from the group consisting of K, T and Y; 2 represents the amino acid residue N or M; 3 represents the amino acid residue T or F; 4 represents the amino acid residue S or A; 5 represents the amino acid residue H or R; 6 represents an amino acid residue selected from the group consisting of A, Y, H and N; and 7 represents the amino acid residue A or T.

In another embodiment, the ankyrin repeat domain comprises a repeat module with the ankyrin repeat sequence motif
1D23G4TPLHLAA56GH7EIVEVLLK8GADVNA (SEQ ID NO: 14)
wherein 1 represents an amino acid residue selected from the group consisting of A, N, R, V, Y, E, H, I, K, L, Q, S and T; in another embodiment, 1 is A, N, R, V or Y; in another embodiment 1 is A or R; 2 represents an amino acid residue selected from the group consisting of S, A, N, R, D, F, L, P, T and Y; in another embodiment, 2 is S, A, N or R; 3 represents an amino acid residue selected from the group consisting of T, V, S, A, L and F; in another embodiment, 3 is T, V, S, A or L; in another embodiment, 3 is T, V or S; 4 represents an amino acid residue selected from the group consisting of W, F and H; 5 represents an amino acid residue selected from the group consisting of P, I, A, L, S, T, V and Y; in another embodiment, 5 is P or I; 6 represents an amino acid residue selected from the group consisting of W, F, I, L, T and V; 7 represents the amino acid residue L or P; and 8 represents an amino acid residue selected from the group consisting of A, H, N and Y.

In another embodiment, the ankyrin repeat domain comprises a repeat module with the ankyrin repeat sequence motif
1D23G4TPLHLAA56GHLEIVEVLLK7GADVNA (SEQ ID NO: 15)
wherein 1 represents an amino acid residue selected from the group consisting of H, Q, A, K, R, D, I, L, M, N, V and Y; in another embodiment, 1 is H, Q, A, K or R; in another embodiment, 1 is A or R; 2 represents an amino acid residue selected from the group consisting of Y, F and H; 3 represents an amino acid residue selected from the group consisting of Q, F and T; 4 represents an amino acid residue selected from the group consisting of W, M, G, H, N and T; preferably W and M; 5 represents an amino acid residue selected from the group consisting of T, A, M, L and V; in another embodiment, 5 is T, A or M; 6 represents an amino acid residue selected from the group consisting of I, L, V, D and T; in another embodiment, 6 is I, L or V; and 7 represents an amino acid residue selected from the group consisting of A, H, N and Y.

In another embodiment, the ankyrin repeat domain comprises a repeat module with the ankyrin repeat sequence motif
1D23GWTPLHLAA45GHLEIVEVLLK6GADVNA (SEQ ID NO: 16)
wherein 1 represents an amino acid residue selected from the group consisting of K, M, N, R and V; 2 represents an amino acid residue selected from the group consisting of Y, H, M and V; 3 represents an amino acid residue selected from the group consisting of F, L, M and V; 4 represents an amino acid residue selected from the group consisting of R, H, V, A, K and N; preferably R, H, V and A; 5 represents an amino acid residue selected from the group consisting of F, D, H, T, Y, M and K; in another embodiment, 5 is F, D, H, T or Y; and 6 represents an amino acid residue selected from the group consisting of A, H, N and Y.

In another embodiment, the ankyrin repeat domain comprises a repeat module with the ankyrin repeat sequence motif
1D23G4TPLHLAA56GHLEIVEVLLK7GADVN8 (SEQ ID NO: 17)
wherein 1 represents an amino acid residue selected from the group consisting of T, A, H, I, N and S; 2 represents an amino acid residue selected from the group consisting of F, I, Q, R, V and N; 3 represents an amino acid residue selected from the group consisting of A, G, N, Q and V; 4 represents the amino acid residue W or Y; 5 represents an amino acid residue selected from the group consisting of A, S, T and M; 6 represents an amino acid residue selected from the group consisting of N, V, S, F, M and W; 7 represents an amino acid residue selected from the group consisting of A, H, N and Y; and 8 represents the amino acid residue T or A.

In another embodiment, the ankyrin repeat domain comprises a repeat module with the ankyrin repeat sequence motif
1D23G4TPLHL5A67GHLEIVEVLLK8GADVNA (SEQ ID NO: 18)
wherein 1 represents an amino acid residue selected from the group consisting of K, A, V and N; 2 represents an amino acid residue selected from the group consisting of N, I and Y; 3 represents an amino acid residue selected from the group consisting of T, F, Y and W; 4 represents an amino acid residue selected from the group consisting of W, D and Y; 5 represents the amino acid residue S or A; 6 represents an amino acid residue selected from the group consisting of D, I and M; 7 represents an amino acid residue selected from the group consisting of L, T and Y; and 8 represents an amino acid residue selected from the group consisting of A, H, Y and N.

In another embodiment, the ankyrin repeat domain comprises a repeat module with the ankyrin repeat sequence motif
1DFK2DTPLHLAA34GH5EIVEVLLK6GADVNA (SEQ ID NO: 19)
wherein 1 represents an amino acid residue selected from the group consisting of L, S and T; 2 represents an amino acid residue selected from the group consisting of G, S and C; in another embodiment, 2 is G or S; 3 represents the amino acid residue S or A; 4 represents an amino acid residue selected from the group consisting of Q, S, M and N; in another embodiment, 4 is Q or S; 5 represents an amino acid residue selected from the group consisting of L, M and Q; and 6 represents an amino acid residue selected from the group consisting of A, H, N, Y and D.

In another embodiment, the ankyrin repeat domain comprises a repeat module with the ankyrin repeat sequence motif
1D2L34TPLHLA567GHLEIVEVLLK8GADVNA (SEQ ID NO: 20)
wherein 1 represents an amino acid residue selected from the group consisting of Y, H, F, I, L and W; preferably Y and H; 2 represents an amino acid residue selected from the group consisting of M, D, I, L, V; in another embodiment, 2 is M or D; 3 represents an amino acid residue selected from the group consisting of G, S and V; 4 represents the amino acid residue W or F; 5 represents an amino acid residue selected from the group consisting of A, G and T; 6 represents the amino acid residue D or W; 7 represents the amino acid residue L or F; and 8 represents an amino acid residue selected from the group consisting of A, H, N and Y.

In another embodiment, the ankyrin repeat domain comprises a repeat module with the ankyrin repeat sequence motif
1D23G4TPL5LAA67GHLEIVEVLLK8GADVNA (SEQ ID NO: 21)
wherein 1 represents an amino acid residue selected from the group consisting of K, S, I, N, T and V; in another embodiment, 1 is K or S; 2 represents an amino acid residue selected from the group consisting of K, N, W, A, H, M, Q and S; preferably K and N; 3 represents an amino acid residue selected from the group consisting of F, Q, L, H and V; preferably F, Q and L; 4 represents the amino acid residue F or T; 5 represents the amino acid residue Q or H; 6 represents the amino acid residue Y or S; 7 represents an amino acid residue selected from the group consisting of N, H, Y and M; in another embodiment, 7 is N or H; and 8 represents an amino acid residue selected from the group consisting of A, H, N and Y.

In another embodiment, the ankyrin repeat domain comprises a repeat module with the ankyrin repeat sequence motif
1D23GWT4LHLAADLG5LEIVEVLLK6GADVNA (SEQ ID NO: 22)
wherein 1 represents an amino acid residue selected from the group consisting of F, Y, H and W; preferably F, Y and H; 2 represents an amino acid residue selected from the group consisting of I, M, D and V; in another embodiment, 2 is I, M or D; 3 represents the amino acid residue F or L; 4 represents the amino acid residue L or P; 5 represents an amino acid residue selected from the group consisting of H, L and Y; and 6 represents an amino acid residue selected from the group consisting of A, H, N, C and Y.

One or more polyethylene glycol moieties may be attached at different positions in the binding protein, and such attachment may be achieved by reaction with amines, thiols or other suitable reactive groups. Attachment of polyethylene glycol moieties (PEGylation) may be site-directed, wherein a suitable reactive group is introduced into the protein to create a site where PEGylation preferentially occurs, or is originally present in the binding protein. The thiol group may be present in a cysteine residue; and the amine group may be, for example, a primary amine found at the N-terminus of the polypeptide or an amine group present in the side chain of an amino acid, such as lysine or arginine. In one embodiment, the binding protein is modified so as to have a cysteine residue at a desired position, permitting site directed PEGylation on the cysteine, for example by reaction with a polyethylene glycol derivative carrying a maleimide function. The polyethylene glycol moiety may vary widely in molecular weight (i.e. from about 1 kDa to about 100 kDa) and may be branched or linear. In one embodiment, the polyethylene glycol has a molecular weight of about 1 to about 50 kDa; in another embodiment, the polyethylene glycol has a molecular weight of about 10 kDa to about 40 kDa, about 15 kDa to about 30 kDa, or about 20 kDa. Examples of such binding proteins and methods for synthesizing them are provided in U.S. Patent No. 8,710,187 (published also as WO 2011/135067), the entire contents of which are incorporated herein by reference.

In one embodiment, one can use a recombinant protein comprising a binding domain as described herein, wherein the recombinant protein is conjugated at its C-terminal cysteine thiol to a maleimide-coupled PEG, such as α-[3-(3-maleimido-1-oxopropyl)amino]propyl-ω-methoxy-polyoxyethylene (NOF, Sunbright ME-200MA (20kD) or Sunbright ME-400MA (40kD)). In one embodiment, one can use a recombinant binding protein comprising a binding domain as described herein, wherein the binding domain is conjugated at its C-terminus via a peptide bond to SEQ ID NO:8, which is in turn conjugated at the C-terminal cysteine thiol to a maleimide-coupled PEG, such as α-[3-(3-maleimido-1-oxopropyl)amino]propyl-ω-methoxy-polyoxyethylene (NOF, Sunbright ME-200MA (20kD) or Sunbright ME-400MA (40kD)).

In some embodiments, the C-terminal cysteine thiol can be conjugated to a pyridyl disulfide-coupled PEG, a vinyl sulfone-coupled PEG, or a PEG coupled via other thiol reagent.

In some embodiments, the PEG and appropriate linking compound has a molecular weight of at least about 2 kD, 5 kD, 10 kD, 20 kD, 30 kD, 40 kD, 50 kD, 70 kD, or 100 kD. In one embodiment the maleimide-coupled PEG has a molecular weight of at least about 2 kD, 5 kD, 10 kD, 20 kD, 30 kD, 40 kD, 50 kD, 70 kD, or 100 kD.

In certain embodiments the α-[3-(3-maleimido-1-oxopropyl)amino]propyl-ω-methoxy-polyoxyethylene has a molecular weight of at least about 20 kD or at least about 40 kD.

In some embodiments, a recombinant binding protein can be conjugated at a N-terminal amino group to a suitable PEG-containing linking compound. In such embodiments, PEG-ethylene reagents, PEG NHS-esters, PEG NHS-carbonate, PEG-p-nitrophenyl carbonates, PEG-triazine reagents, and the like may be conjugated at the N-terminus of a suitable binding protein described herein.

In a further embodiment, the invention relates to nucleic acid molecules encoding the particular recombinant binding proteins. Further, a vector comprising the nucleic acid molecule is considered.

Further, a pharmaceutical composition comprising one or more of the above mentioned binding proteins, in particular recombinant binding proteins comprising repeat domains, or nucleic acid molecules encoding the particular recombinant binding proteins, and optionally a pharmaceutical acceptable carrier and/or diluent is considered.

### Formulation

Pharmaceutical acceptable carriers and/or diluents are known to the person skilled in the art and are explained in more detail below. Even further, a diagnostic composition comprising one or more of the above mentioned recombinant binding proteins, in particular binding proteins comprising repeat domains, is considered.

A pharmaceutical composition comprises binding proteins as described above and a pharmaceutically acceptable carrier, excipient or stabilizer (Remington's Pharmaceutical Sciences 1sixth edition, Osol, A. Ed. [1980]). Suitable carriers, excipients or stabilizers known to the skilled man are saline, Ringer's solution, dextrose solution, Hank's solution, fixed oils, ethyl oleate, 5% dextrose in saline, substances that enhance isotonicity and chemical stability, buffers and preservatives. Other suitable carriers include any carrier that does not itself induce the production of antibodies harmful to the individual receiving the composition such as proteins, polysaccharides, polylactic acids, polyglycolic acids, polymeric amino acids and amino acid copolymers. A pharmaceutical composition may also be a combination formulation, comprising an additional active agent, such as an anticancer agent or an anti-angiogenic agent (for example human VEGF-Axxxb; preferably, human VEGF-A165b).

In one embodiment, a formulation comprises binding proteins as described above and a detergent such as nonionic detergent, including but not limited to polysorbate 20 0.04%, a buffer such as histidine, phosphate or lactic acid and a sugar such as sucrose or trehalose. The formulation may also comprise PBS.

The formulations to be used for in vivo administration must be aseptic or sterile. This is readily accomplished by filtration through sterile filtration membranes.

### Methods of treatment

In one embodiment, the method of the invention comprises a method of inhibiting binding between VEGF-Axxx and VEGFR-2 by administering to a patient in need of such inhibition, a dose of about 0.25 mg to about 4 mg of a recombinant binding protein comprising an ankyrin repeat domain, wherein the ankyrin repeat domain is selected from the group consisting of the ankyrin repeat domains of SEQ ID NOS:1 to 7.

In some embodiments, the ankyrin repeat domain is selected from the group consisting of the ankyrin repeat domains of SEQ ID NOS:1 to 5. In some embodiments, the ankyrin repeat domain is selected from the group consisting of the ankyrin repeat domains of SEQ ID NOS: 2 or 3. In one embodiment, the recombinant binding domain binds VEGF-Axxx with a Kd below 10⁹M.

The methods can be used to treat certain ocular conditions, including those related to ischemic retinopathy, neovascular retinopathy, or both ischemic retinopathy and neovascular retinopathy. Some conditions related to ischemic retinopathy, that can be treated by methods disclosed herein, can include diabetic macular edema, central vein occlusion, and branched vein occlusion. Some conditions related to neovascular retinopathy, that can be treated by methods disclosed herein, can include proliferative diabetic retinopathy, exudative age-related macular degeneration, pathological myopia, choroidal neovascularation, neovascularization secondary to histoplasmosis, polypoidal choroidal neovasularization, and retinal angiomatous proliferation, The method may be used to treat age-related macular degeneration, diabetic macular edema, pathological myopia branch retinal vein occlusion, and central retinal vein occlusion. The method may also be used to treat patients have the above-listed diseases who are refractory to existing anti-VEGF therapies.

To "treat," as used here, means to deal with medically. It includes, for example, administering the recombinant binding protein of the invention to prevent the onset of AMD as well as to alleviate its severity.

Macular degeneration results from the neovascular growth of the choroid vessel underneath the macula. There are two types of advanced macular degeneration: exudative and atrophic. While exudative macular degeneration only comprises 15% of all macular degeneration, nearly all exudative macular degeneration leads to blindness. Once one eye is affected by exudative macular degeneration, the condition almost always affects the other eye. Exudative and atrophic macular degeneration are often called age-related macular degeneration or age-related "wet" macular degeneration as the diseases are found mostly in elderly persons.

As used here, "refractory to anti-VEGF therapy" refers to the inability to achieve a satisfactory physiological response with known anti-VEGF therapy, such as ranibizumab, bevacizumab, aflibercept, or pegaptanib therapy. Such patients may, for example, have less than a 20% decrease in abnormal central retina thickness (center 1mm² area of the macula) after at least 3 intravitreal injections of ranibizumab, bevacizumab, or aflibercept (or at least 3 intravitreal injections of a combination of any of the foregoing therapies). In one embodiment, a patient who is refractory to anti-VEGF therapy experiences a continuing worsening of vision despite ranibizumab, bevacizumab, aflibercept, or pegaptanib therapy. In another embodiment, a patient who is refractory to anti-VEGF therapy experiences thickening of the retina despite ranibizumab, bevacizumab, aflibercept, or pegaptanib therapy. In some embodiments, patients refractory to anti-VEGF therapy demonstrate negligible anatomical improvement despite receiving ranibizumab, bevacizumab, aflibercept, or pegaptanib therapy.

In some embodiments, the binding proteins are administered intravitreally at a dose between about 0.1 mg and about 10 mg of binding proteins per injection. In some embodiments, the binding proteins are administered at a dose of between about 0.25 mg and about 5 mg of binding proteins per injection, between about 0.25 mg and about 4 mg of binding proteins per injection, between about 0.25 mg and about 3 mg binding proteins per injection, between about 0.25 mg and about 2 mg binding proteins per injection, and between about 0.25 mg and about 1 mg binding proteins per injection; in other embodiments, the binding proteins are administered at a dose of between about 0.5 mg and about 5 mg of binding proteins per injection, between about 0.5 mg and about 4 mg of binding proteins per injection, between about 0.5 mg and about 3 mg binding proteins per injection, between about 0.5 mg and about 2 mg binding proteins per injection, and between about 0.5 mg and about 1 mg binding proteins per injection; in other embodiments, the binding proteins are administered at a dose of between about 1 mg and about 5 mg of binding proteins per injection, between about 1 mg and about 4 mg of binding proteins per injection, between about 1 mg and about 3 mg binding proteins per injection, and between about 1 mg and about 2 mg binding proteins per injection.

In one embodiment, the binding proteins are administered at a dose of about 0.1 mg, about 0.2 mg, about 0.25 mg, about 0.3 mg, about 0.35 mg, about 0.4 mg, about 0.45 mg, about 0.5 mg, about 0.55 mg, about 0.6 mg, about 0.65 mg, about 0.7 mg, about 0.75 mg, about 0.8 mg, about 0.9 mg, about 0.95 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2 mg, about 2.0 mg, 2.1 mg, about 2.2 mg, about 2.3 mg, about 2.4 mg, about 2.5 mg, about 2.6 mg, about 2.7 mg, about 2.8 mg, about 2.9 mg, about 3 mg, about 3.0 mg, 3.1 mg, about 3.2 mg, about 3.3 mg, about 3.4 mg, about 3.5 mg, about 3.6 mg, about 3.7 mg, about 3.8 mg, about 3.9 mg, about 4 mg, about 4.0 mg, 4.1 mg, about 4.2 mg, about 4.3 mg, about 4.4 mg, about 4.5 mg, about 4.6 mg, about 4.7 mg, about 4.8 mg, about 4.9 mg, about 5 mg, and about 5.0 mg of binding proteins per injection.

In one embodiment, the binding protein is administered in an initial dose of 2 to 5 doses, with an interval of 25 to 35 days between each dose; that is, a single dose of the binding protein is administered to an eye of a patient, then another dose is administered 25 to 35 days later to the same eye, with the doses repeated, if necessary, until the eye receives a total of 2 to 5 initial doses.

In one embodiment, the binding protein is first administered in 2 doses, with an interval of 25 to 35 days between each dose. In this embodiment, a single dose of the binding protein (for example, a single injection of 0.5 mg to about 4 mg of binding protein) is administered to an eye, and then the same dose is administered to the eye 25 to 35 days later, for a total of 2 initial doses per treated eye.

In one embodiment, the binding protein is first administered in 3 doses, with an interval of 25 to 35 days between each dose. In this embodiment, a single dose of the binding protein (for example, a single injection of 0.5 mg to about 4 mg of binding protein) is administered to an eye, the same dose is administered to the eye 25 to 35 days later, and then is administered again 25 to 35 days later, for a total of 3 initial doses per treated eye.

In one embodiment, the binding protein is first administered in 4 doses, with an interval of 25 to 35 days between each dose. In this embodiment, a single dose of the binding protein (for example, a single injection of 0.5 mg to about 4 mg of binding protein) is administered to an eye, the same dose is administered to the eye 25 to 35 days later, is administered again 25 to 35 days later, and then is administered yet again 25 to 35 days later, for a total of 4 initial doses per treated eye.

In one embodiment, the binding protein is first administered in 5 doses, with an interval of 25 to 35 days between each dose. In this embodiment, a single dose of the binding protein (for example, a single injection of 0.5 mg to about 4 mg of binding protein) is administered to an eye, the same dose is administered to the eye 25 to 35 days later, is administered again 25 to 35 days later, and is administered yet again 25 to 35 days later, and then is administered once more 25 to 35 days later, for a total of 5 initial doses per treated eye.

In another embodiment, the binding protein is first administered to an eye in 2 to 5 initial doses, with an interval of 25 to 35 days between each dose, and then is followed by at least one additional dose, with an interval of 50 to 130 days between each additional dose.

In one embodiment, there is an interval of 50 to 100 days, 50 to 95 days, 50 to 90 days, 50 to 85 days, 50 to 80 days, 50 to 75 days, 50 to 70 days, 50 to 65 days, and 50 to 60 days between each additional dose; in another embodiment, there is an interval of 55 to 100 days, 55 to 95 days, 55 to 90 days, 55 to 85 days, 55 to 80 days, 55 to 75 days, 55 to 70 days, 55 to 65 days, and 55 to 60 days between each additional dose; in another embodiment, there is an interval of 60 to 100 days, 60 to 95 days, 60 to 90 days, 60 to 85 days, 60 to 80 days, 60 to 75 days, 60 to 70 days, and 60 to 65 days between each additional dose; in another embodiment, there is an interval of 65 to 100 days, 65 to 95 days, 65 to 90 days, 65 to 85 days, 65 to 80 days, 65 to 75 days, and 65 to 70 days between each additional dose; in another embodiment, there is an interval of 70 to 100 days, 70 to 95 days, 70 to 90 days, 70 to 85 days, 70 to 80 days, and 70 to 75 days between each additional dose; in another embodiment, there is an interval of 75 to 100 days, 75 to 95 days, 75 to 90 days, 75 to 85 days, and 75 to 80 days between each additional dose; in another embodiment, there is an interval of 80 to 100 days, 80 to 95 days, 80 to 90 days, and 80 to 85 days between each additional dose; in another embodiment, there is an interval of 85 to 100 days, 85 to 95 days, and 85 to 90 days between each additional dose; in another embodiment, there is an interval of 90 to 100 days, and 90 to 95 days between each additional dose. In another embodiment, there is an interval of 30, 35, 40, 45, 50, 55, 60, 65, 70, 75, 80, 85, 90, 95, 100, 105, 110, 115, 120, 125, or 130 days between each additional dose.

For example, in one embodiment, a single dose of the binding protein (such as a single injection of 0.5 mg to about 4 mg of binding protein) is administered to an eye, the same dose is administered to the eye 25 to 35 days later, and then is administered again 25 to 35 days later, for a total of 3 initial doses per treated eye, whereupon the same dose is then administered to the eye every 55 to 65 days, for as long as the eye requires treatment.

According to some embodiments, a patient receiving injections of recombinant binding proteins according to the method of the invention can demonstrate a 20% or greater reduction, 25% or greater reduction, 30% or greater reduction, 35% or greater reduction, 40% or greater reduction, 45% or greater reduction, 50% or greater reduction, 55% or greater reduction, 60% or greater reduction, 65% or greater reduction, 70% or greater reduction, 75% or greater reduction, 80% or greater reduction, 85% or greater reduction, 90% or greater reduction, 95% or greater reduction, 99% reduction, or 100% reduction in abnormal central retina thickness from baseline.

According to some embodiments, a patient who is refractory to Anti-VEGF treatments such as ranibizumab, bevacizumab, aflibercept, or pegaptanib, after receiving one or more injections of binding proteins disclosed herein can demonstrate about a 20% or greater reduction in abnormal central retina thickness from baseline. According to some embodiments a patient after receiving one or more injections of binding proteins disclosed herein can demonstrate about a 30% or greater reduction in abnormal central retina thickness from baseline. According to yet other embodiments a patient after receiving one or more injections of binding proteins disclosed herein can demonstrate less than a 40% or greater reduction in abnormal central retina thickness from baseline.

For example, a method for the treatment of an ocular condition, such as age-related macular degeneration can include an intravitreal injection of a recombinant binding protein in an amount in the range of 0.5 mg to 5 mg at the frequencies described herein. The recombinant binding protein comprises the ankyrin repeat domains of SEQ ID NO: 3.

In another example, a method for the treatment of age-related macular degeneration in a patient who is refractory to an anti-VEGF treatment such as ranibizumab, bevacizumab, or ranibizumab and bevacizumab, can include an intravitreal injection of a recombinant binding protein in an amount in the range of 0.5 mg to 5 mg. The recombinant binding protein comprises the ankyrin repeat domains of SEQ ID NO: 3. In such an example, a patient receiving such injection or injections can experience more than a 20% decrease in abnormal central retina thickness after receiving one or more injections. In some embodiments, a patient receiving such injection or injections does not experience a thickening of the retina after receiving one, two, three, four, five, or more injections.

In another example, a method for the treatment of diabetic macular edema in a patient who is refractory to an anti-VEGF treatment such as ranibizumab, bevacizumab, or ranibizumab and bevacizumab, can include an intravitreal injection of a recombinant binding protein in an amount in the range of 0.5 mg to 5 mg. The recombinant binding comprises the ankyrin repeat domain of SEQ ID NO: 3. In such an example, a patient receiving such injection or injections can experience more than a 20% decrease in abnormal central retina thickness after receiving one, two, three, four, five, or more injections. In some embodiments, a patient receiving such injection or injections does not experience a thickening of the retina after receiving one, two, three, four, five, or more injections.

### Examples

The invention is illustrated further by the following examples. The inventors tested abicipar pegol, a binding protein of the invention comprising an ankyrin repeat domain of SEQ ID NO:3, conjugated to polyethylene glycol (the compound has the generic name "abicipar pegol," occasionally referred to herein as simply "abicipar").

### Overview

The inventors had previously tested abicipar pegol in an open-label, dose-escalation assessment of safety administered as an intravitreal injection to patients with advanced exudative AMD who were refractory to anti-VEGF therapy. They followed that study with a randomized, double-masked evaluation of abicipar and ranibizumab in treatment-naive patients with exudative AMD ("the Stage 2 study"). The objectives were to assess the safety and duration of treatment effects with 4.2 mg and 3mg abicipar on retinal edema and best-corrected visual acuity (BCVA) and to characterize the systemic pharmacokinetic profile of abicipar.

In this example, the inventors performed a randomized, double-masked comparison of abicipar and ranibizumab ("the Stage 3 study"). Patients were randomized to one of three groups, abicipar 1 mg, abicipar 2 mg or ranabizumab 0.5 mg, and were followed for 20 weeks. All patients received 3 monthly doses at weeks 0, 4, and 8. Ranabizumab-treated patients received additional ranibizumab doses at weeks 12 and 16, while abicipar patients received sham injections. The goal of Stage 3 was to assess whether similar or better effects on BCVA and retina edema could be achieved with fewer doses of abicipar (3 doses) vs ranibizumab (5 doses); comparisons were made 8 and 12 weeks after the final dose of abicipar vs 4 weeks after the fourth and fifth doses of ranibizumab.

The primary efficacy variable in Stage 3 was the mean change from baseline in BCVA in the study eye at week 16 (8 weeks after the third injection). Patients treated with 2 mg and 1 mg abicipar gained 8.2 and 6.3 letters, respectively, compared with the ranibizumab-treated patients who gained 5.3 letters (4 weeks after the fourth injection). A key secondary efficacy variable was the elimination of intraretinal fluid. Intraverinal fluid was characterized as intraretinal edema, intraretinal cysts and subretinal fluid as assessed with optical coherence tomography. At week 12, 4 weeks after the third dose of treatment, the proportion of patients with no intraretinal fluid was 78%, 68 and 50% in the abicipar 2 mg, abicipar 1 mg and ranibizumab groups. At week 16, 8 weeks after the third dose of abicipar and 4 weeks after the fourth dose of ranibizumab, the proportion of patients with no intraretinal fluid was 52, 44 and 19% in the abicipar 2 mg, abicipar 1 mg and ranibizumab groups respectively. The presence of intraretinal fluid causes a disruption of the neural network in the retina leading to vision loss. Elimination of intraretinal fluid preserves vision.

### Patient selection

Approximately 64 patients were randomized in a 3:3:2 allocation to one of the following groups:
- 2 mg abicipar administered at Baseline, week 4 and week 8 (3 injections)
- 1 mg abicipar administered at Baseline, week 4 and week 8 (3 injections)
- 0.5 mg ranibizumab administered at Baseline, weeks 4, 8, 12, and 16 (5 injections)

Sixty four patients were selected. These had a mean age of 76.6 years, were predominantly Caucasian, and the majority were female. At baseline, mean BCVA ranged from 58.5 and 60.4 letters (∼20/63 Snellen equivalent). Mean baseline central retinal thickness ("CRT") ranged between 463 and 526 µm. There were no significant differences between the treatment groups for any of the demographic or baseline characteristics (Table 1), although the proportion of patients with predominantly classic lesion was markedly higher in the abicipar 2.0 mg group than in the other treatment groups.

BCVA was quantified by using a modification of the ETDRS method as described in Arch Ophthalmol., 119(10):1417-36 (2001). BCVA testing preceded any examination requiring contact with the eye, and was performed following refraction.

Refraction was performed at 4 meters, unless the visual acuity was reduced, in which case it was performed at 1 meter. The right eye was refracted first, followed by the left eye. A trial frame was placed on the patient's face and adjusted so that the lens cells were parallel to the anterior plane of the orbit and centered in front of the pupils. The lens cells were adjusted for the proper distance from the cornea. The eye not being refracted was occluded by patching with folded tissue and applying a black occluder over the eye. The lens correction obtained from a beginning, approximate refraction was inserted into the trial frame. The lenses were positioned by inserting them in the compartment closest to the eye; the cylindrical lens correction was placed in the compartment in front of the spherical correction and the axis was adjusted. Patients were encouraged to use eccentric viewing, if necessary, making certain that the other eye was completely occluded. Sphere power, cylinder axes, and cylinder power were then determined and refined.

BVCA testing began at 4 meters. First, the right eye was tested and then the left eye was tested. The distance from the participant's eyes to the visual acuity chart was 4 meters (13 feet and 1.5 inches, or 157.5 inches). The participant was allowed to stand or sit; in either case, the examiner ensured that the participant was standing or sitting comfortably, that the head did not move forward or backward during the test, and that the participant's eyes remained at the 4-meter distance. The examiner scored each letter the participant read as right or wrong. Once a participant had identified a letter with a definite single-letter response and read the next letter, a correction of the previous letter was not accepted. If the participant changed a response aloud (e.g., "That was a 'C,' not an 'O'") before he has read aloud the next letter, then the change was accepted. If the participant changed the response after beginning to read the next letter, the change was not accepted. When the participant said he could not read a letter, he was encouraged to guess. If the participant identified a letter as one of two or more letters, he was asked to choose one letter and, if necessary, to guess even if he read the next letter. When it became evident that no further meaningful readings could be made (usually with the participant being unable to guess at a letter), despite urgings to read or guess, the examiner stopped the test for that eye. There were several reasons for encouraging participants to guess: participants' statements that they cannot identify a letter are often unreliable; encouraging them to guess helps to maximize the participant's effort; it helps to assure uniformity among procedures performed in different clinics; and it may help to prevent participant bias (malingering).

Eyes reading 19 or fewer letters correctly at 4 meters were tested at 1 meter. Before testing at 1 meter, a +0.75 sphere was added to the 4-meter correction already in the trial frame to compensate for the closer testing distance. While the participant could stand or sit for the 4-meter test, the participant could only sit for the 1-meter test. The participant was asked to read only the first 6 lines at 1 meter, making 30 the maximum score at that distance.

After the test of the right eye was completed, the test was repeated for the left eye, starting at 4 meters.

**Table 1: Demographics and Baseline Characteristics (Stage 3, mITT)**

| | **ABICIPAR 2 MG** (N=23) | **ABICIPAR 1 MG** (N=25) | **RANIBIZUMAB 0.5 MG** (N=16) |
|---|---|---|---|
| Age (years) | 77.9 | 75.5 | 76.5 |
| Sex (% female) | 56.5 | 72.0 | 50.0 |
| Race (% Caucasian) | 95.7 | 96.0 | 100 |
| Mean Baseline BCVA (letters) | 58.5 | 58.4 | 60.4 |
| Mean Baseline Retinal Thickness (µm) | 466.0 | 526.1 | 463.3 |
| Lesion type | | | |
| Predominant classic | 44.0% | 26.1% | 25.0% |
| Occult | 52.0% | 69.6% | 68.8% |

### Key Safety Variables

- Adverse Events (AEs) and Serious Adverse Events (SAEs)
- BCVA
- General physical exam
- Blood chemistry, hematology and urinalysis
- Post-injection evaluation

### Immunogenicity and Systemic Pharmacokinetic Measures:

- Systemic (serum) levels of abicipar
- Binding antibodies and neutralizing antibodies to abicipar

### Efficacy Measures

- BCVA assessed with the Early Treatment of Diabetic Retinopathy Study (ETDRS) visual acuity protocol
   ∘ Mean change from baseline
   ∘ BCVA responder analysis:
      1. ≥15 letter improvement from baseline (improved vision)
      2. less than a 15 letter loss from baseline (stable vision)
- Retinal edema or central retinal thickness (center 1 mm² area of the fovea; CRT) as measured by optical coherence tomography (OCT) and evaluated by a central reading center (CRC)
- Evaluation of intraretinal fluid, intraretinal cysts, and subretinal fluid by OCT as measured by the CRC.

OCT is a laser-based, non-invasive, diagnostic system providing high-resolution imaging optical sections of the retina. The thickness of the central 1 mm subfield of the retina in the study eye was captured using either a Spectralis Spectral Domain OCT (SD-OCT) by Heidelberg Engineering or a Cirrus Spectral Domain OCT by Carl Zeiss. The Spectralis OCT device captured a volume scan with 512 A scans and 49 B scans at 6.0 mm x 6.0 mm and a cross hair scan with horizontal and vertical B scans at the 6.0 mm scan length and 768 resolution with fixation on the macula. The device was run with Heidelberg Eye Explorer Version 1.6.2.0 and HRA2 / Spectralis Family Acquisition Module 5.1.2.0 or higher version. The Cirrus OCT device captured a macular cube scan with 512 A scans and 128 B scans at 6.0 mm x 6.0 mm and a cross hair scan with horizontal and vertical B-scans at the 6.0 mm scan length and 1024 A scans with fixation on the macula. The device was running Cirrus software version 4.5 or higher.

Electronic OCT images collected from patients at the screening visit were reviewed by a CRC to confirm patient eligibility. Electronic OCT images collected from all patients at all of the study visits was sent to the CRC to assess the effect of treatment on the resolution of retinal fluid.

The same OCT system (i.e., Spectralis or Cirrus) was used to evaluate CRT for any given patient throughout the study.

Data used to ascertain the disease recurrence include BCVA and CRT graded by the central reading center for the study eye. New hemorrhages or existing hemorrhage of greater than 1.25 mm² as evaluated by the investigator were captured on the retreatment case report form (CRF). A positive response captured on this CRF was used as a confirmation of new or increase of an existing hemorrhage.

Abicipar-treated patients could escape to standard of care therapy (SOC) if there was evidence of active disease after the third injection of study medication (week 12); ranibizumab-treated patients received repeat injections at 4-week intervals regardless of evidence of active disease.

### Evidence of Active Disease

The inventors' protocol provided that, at week 12 and beyond, eyes with retinal fluid on OCT should be treated with the exception of eyes in which there has been no decrease in retinal fluid after 3 consecutive injections administered at 4-week intervals. For such eyes, it is possible that continued treatment may be futile and the Ophthalmologist and patient may choose to suspend treatment. Treatment may be reinstituted in these eyes at a later visit if there is increased retinal fluid (relative to the visit when treatment was stopped) on OCT. If there is no retinal fluid on OCT, but there are other signs of active CNV, the eye should be treated. These signs include new subretinal or intraretinal hemorrhage, persistent subretinal or intraretinal hemorrhage, decreased visual acuity relative to the last visit without another explanation, increased lesion size on fluorescein angiography (FA) relative to the last angiogram, or leakage on FA.

### Key Follow-up Visits

- Stage 1: Day 3, weeks 1, 2, 4, 8, 12, 16, 20, 24/exit
- Stage 2: Day 3, weeks 1, 2, 4, 6, 8, 12, 16, 20, 24, 28 and 32/exit
- Stage 3: Day 3, weeks 1, 4, 8, 12, 16, 20/exit

### Analyses

The safety population was analyzed for adverse events and the modified intenttreat (mITT) population was analyzed for disposition, demographics, baseline characteristics, central retinal thickness, and BCVA; CRT and BCVA last-observation-carried-forward (LOCF) was used to input data following the patient receiving treatment with SOC (eg, ranibizumab, aflibercept, bevacizumab).

### Safety

The most common (≥ 2 events/group) adverse events (AEs) in the study eye regardless of causality, were retinal hemorrhage, vitreous floaters, vitreous detachment and eye pain in the abicipar-groups, and macular scar and retinal hemorrhage in the ranibizumab group (Table 2). There were no serious adverse events reported in any group. Inflammation-related AEs occurred in 2 of the 2 mg-treated patients and in 3 of the 1 mg-treated patients (Table 2). None of the adverse events occurred following the first study injection; the choroiditis and uveitis AEs occurred following the second injection; the iritis and vitritis AEs occurred following the third injection. There were no reports of ocular inflammation in the ranibizumab-treated group.

**Table 2: Adverse Events Regardless of Causality (incidence ≥2 events/group) and Ocular Inflammation (any event) in the Study Eye**

| | **ABICIPAR 2 MG** (N=23) | **ABICIPAR 1 MG** (N=25) | **RANIBIZUMAB 0.5 MG** (N=16) |
|---|---|---|---|
| Macular scar | 0 (0%) | 0 (0%) | 2 (12.5%) |
| Retinal haemorrhage | 0 (0%) | 3 (12.0%) | 2 (12.5%) |
| Vitreous floaters | 1 (4.3%) | 3 (12.0%) | 1 (6.3%) |
| Vitreous detachment | 2 (8.7%) | 2 (8.0%) | 0 (0%) |
| Eye pain | 2 (8.7%) | 1 (4.0%) | 1 (6.3%) |
| Iritis | 1 (4.3%) | 1 (4.0%) | 0 (0%) |
| Choroiditis | 1 (4.3%) | 0 (0%) | 0 (0%) |
| Uveitis | 0 (0%) | 1 (4.0%) | 0 (0%) |
| Vitritis | 0 (0%) | 1 (4.0%) | 0 (0%) |

### Efficacy

In the 2 mg and 1 mg abicipar groups, no patients escaped to SOC until week 12. In the 2 mg group, 3 patients escaped to SOC at week 12, and 5 patients escaped at week 16. In the 1 mg group, 5 patients escaped at week 12, and 6 at week 16. Ranibizumab-treated patients with evidence of active disease after week 12 continued to be retreated with ranibizumab; 6 ranibizumab-treated patients met the criteria for SOC at week 12 and 3 at week 16.

### Best-Corrected Visual Acuity (BCVA)

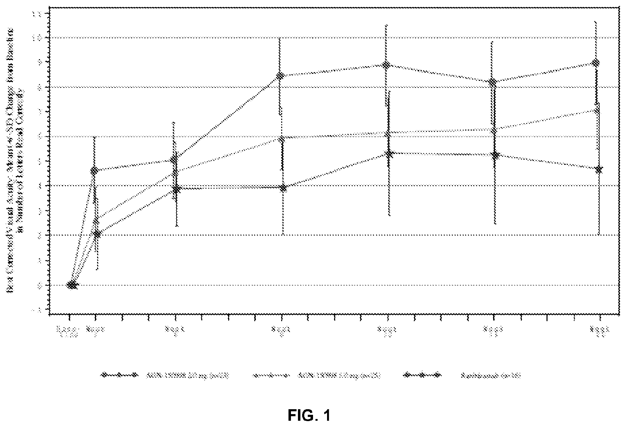

Mean BCVA change from baseline improved in all groups; at week 12, 4 weeks after the last dose of abicipar, the BCVA change from baseline was 8.9, 6.2 and 5.3 letters in the 2 mg, 1 mg and ranibizumab treatment groups, respectively. At week 16, the primary endpoint, the BCVA change from baseline was 8.2, 6.3 and 5.3 letters in the respective groups, as shown in Figure 1. Differences between abicipar and ranibizumab treatment groups were thus considerable, albeit not statistically significant (in Figure 1 and Figures 2, 3, and 4, data after receiving standard-of-care are considered missing; missing values are imputed using LOCF).

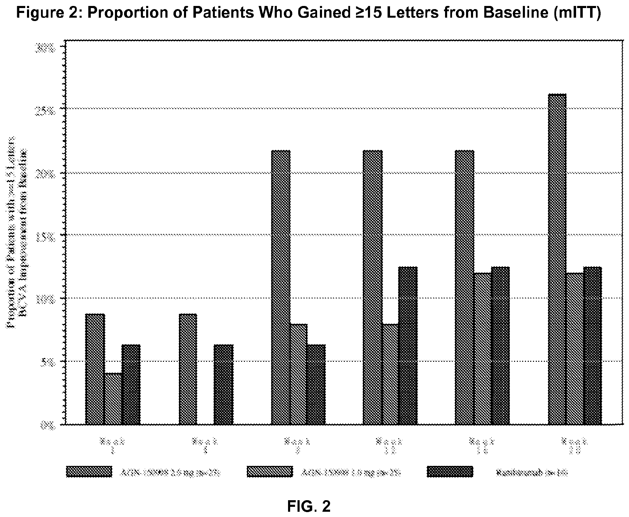

The proportion of patients with a 15 or more letter improvement in BCVA was numerically higher in the 2 mg abicipar group compared to the ranibizumab group. At week 12, 22%, 8% and 13% in the 2 mg, 1 mg abicipar and ranibizumab groups improved by 15 or more letters, respectively. At week 16, the values were 22%, 12% and 13% (Fig. 2). None of the patients in the abicipar groups and 1 patient in the ranibizumab group lost 15 or more letters during the 20-week study.

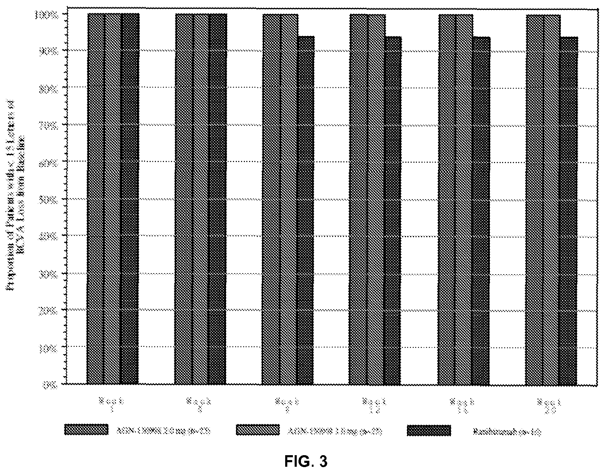

Stable vision was assessed as the loss of BCVA less than 15 letters. All patients in the abicipar groups achieved stable vision; one patient in the ranibizumab group did not (Fig. 3).

### Central Retinal Thickness

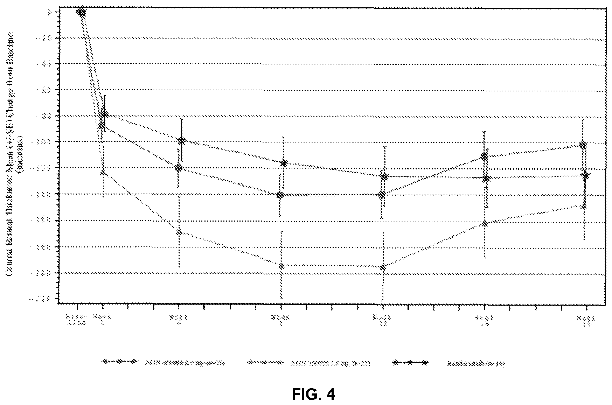

While baseline CRT values were comparable between the 2 mg abicipar and ranibizumab treatment groups, the 1 mg abicipar group was approximately 60 µm thicker. By week 12, CRT reductions were 140, 195 and 126 µm in the three treatment groups, respectively. At week 16, these values were 111, 161, and 127 µm; differences between the 2.0 mg abicipar and ranibizumab treatment groups were not significant at any time. The proportion of patients in whom CRT was reduced to or below the upper limit of normal was numerically higher in the abicipar group at weeks 1 through 16 with the difference at week 8 being significantly greater in the 1 mg group compared with the ranibizumab group (p=0.041). At week 12, the proportion of patients with CRT reduced to or below the upper limit of normal was 57%, 64% and 44% in the three treatment groups, respectively. At week 16 the values were 48%, 52% and 44%. Figure 4 shows the mean CRT values across the study.

### Effects of Treatment on Anatomical Resolution of Fluid

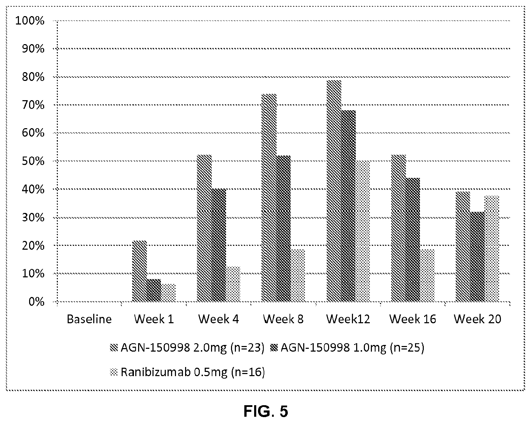

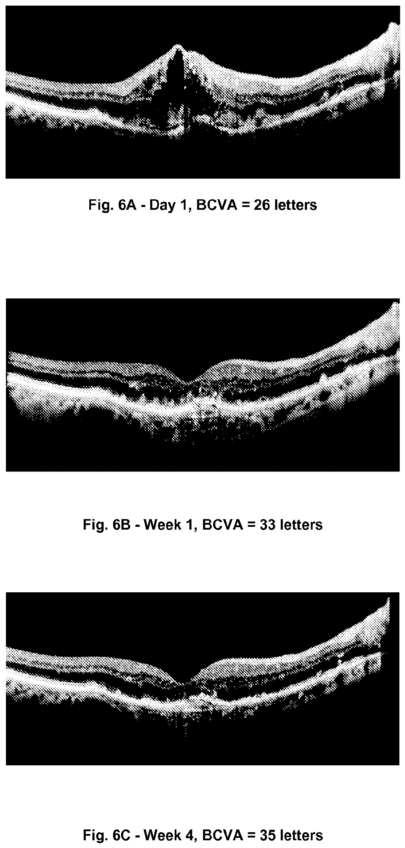

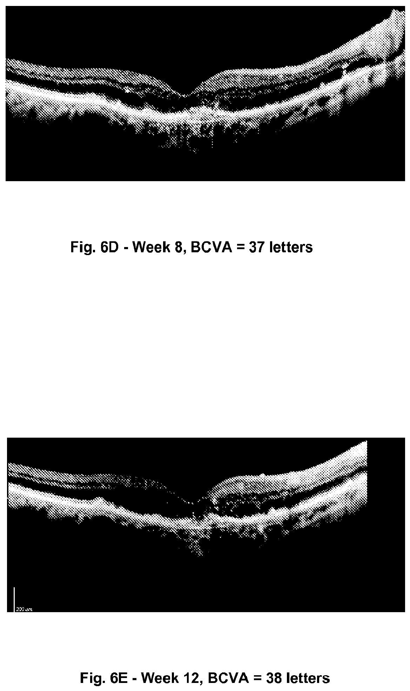

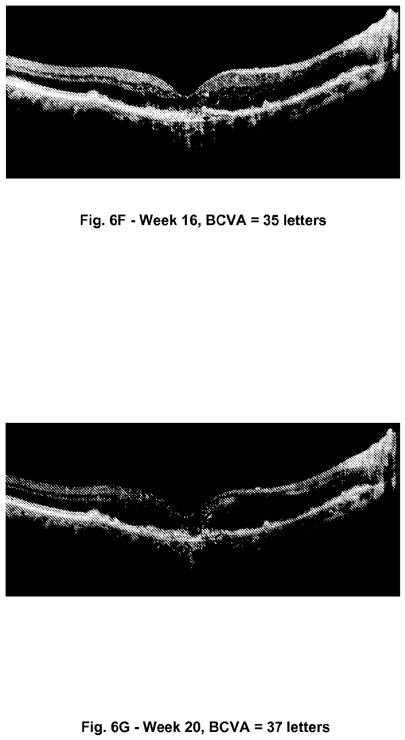

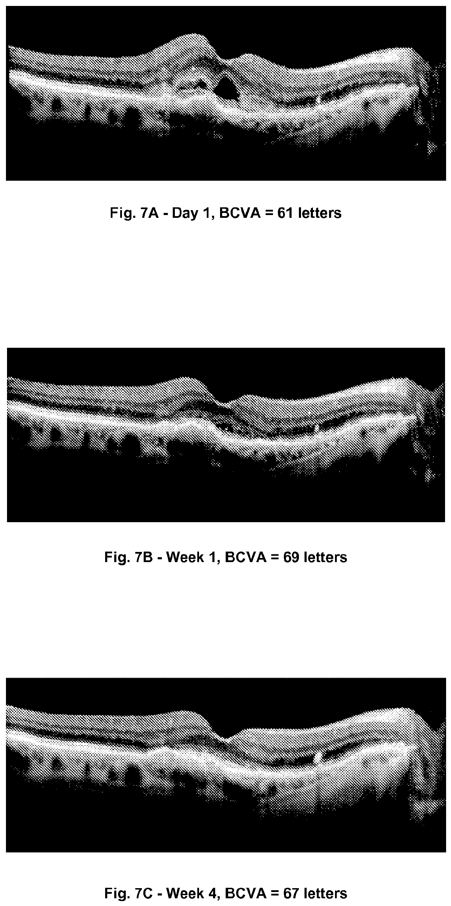

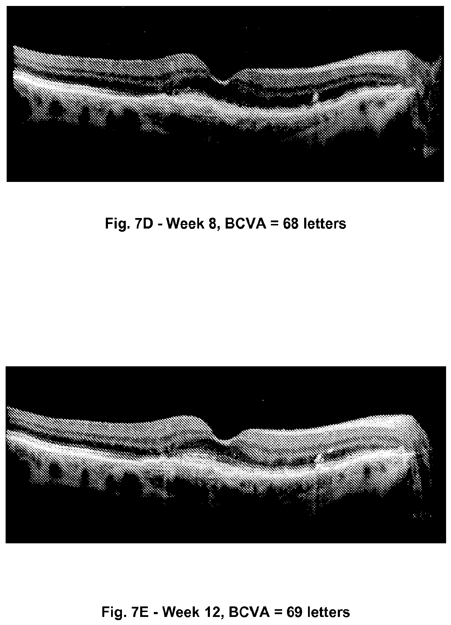

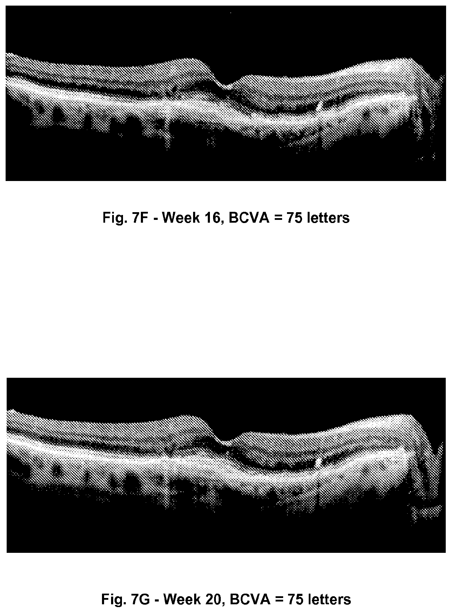

The presence of intraretinal fluid, intraretinal cysts and subretinal fluid in sdOCT images were scored by the Reading Center as measures of active exudative lesions. The proportion of patients in whom all 3 fluid compartments were resolved (All Dry) 4 weeks after the first, second, and third doses was 52, 74,and 78% in the 2 mg abicipar group, 40, 52 and 68% in the 1 mg abicipar group, and 13, 19 and 50% in the ranibizumab group. At week 16, 8 weeks following the third injection of abicipar 2.0 mg and 1.0 mg, and 4 weeks after the fourth injection of ranibizumab, these values were 52, 44 and 19% in the 2 mg, 1 mg abicipar and ranibizumab groups, respectively. Figure 5 shows the proportion of patients in whom intraretinal fluid, intraretinal cysts, and subretinal fluid (all three compartments) resolved after treatment. Two examples, in patients treated with 2 mg abicipar, are shown in Figures 6 and 7. There is a marked reduction in central retinal thickness in these patients, evidenced by the resolution of abnormal fluid in the central retina.

### Comparison to previous findings

That 1.0 and 2.0 mg abicipar may be used to effectively reduce intraretinal fluid, intraretinal cysts, and subretinal fluid after 4, 8, or 12 weeks of beginning treatment is made all the more surprising by the effect of higher doses on such measures of disease progression. In a clinical trial of 175 treatment-naive patients with exudative AMD, 4.2 mg and 3.0 mg abicipar was found to be no more effective after the initial dose than was ranibizumab in resolving retinal fluid. Patients were dosed on day 1 and then at week 16 or earlier if they met certain re-treatment criteria. Figure 8 shows the proportion of patients in whom intraretinal fluid, intraretinal cysts, and subretinal fluid (all three compartments) have resolved; Figure 9 shows the proportion of patients in whom fluid has resolved in one, two, all three, or none of these compartments.

Although this invention has been disclosed in the context of certain embodiments and examples, it will be understood by those skilled in the art that the present invention extends beyond the specifically disclosed embodiments to other alternative embodiments and/or uses of the invention and obvious modifications and equivalents thereof. In addition while the number of variations have been shown and described in detail, other modifications, which are within the scope of this invention, will be readily apparent to those of skill in the art based on this disclosure. It is also contemplated that various combinations or sub-combinations of the specific features and aspects of the embodiments can be made and still fall within the scope. Accordingly, it should be understood that various features and aspects of the disclosed embodiments can be combined with, or substituted for, one another in order to perform varying modes of the disclosed invention. Thus, it is intended that the scope of the present invention herein disclosed should not be limited by the particular disclosed embodiments described above, but should be determined only by a fair reading of the claims.

### Preferred Embodiments

1. A method of inhibiting binding between VEGF-Axxx and VEGFR-2, the method comprising the steps of administering, to a patient in need of such inhibition, about 0.25 mg to about 4 mg of a recombinant binding protein comprising an ankyrin repeat domain, wherein the recombinant binding protein is administered in 2 to 5 doses, with an interval of 25 to 35 days between each dose.
2. A method of treating macular degeneration, the method comprising the step of administering, to a patient in need of such treatment, about 0.25 mg to about 4 mg of a recombinant binding protein comprising an ankyrin repeat domain, wherein the recombinant binding protein is administered in 2 to 5 doses, with an interval of 25 to 35 days between each dose.
3. The method of item 2, wherein the macular degeneration is exudative macular degeneration.
4. A method of improving visual acuity in a patient having a disease of the retina, the method comprising the step of administering, to a patient in need of such improvement, about 0.25 mg to about 4 mg of a recombinant binding protein comprising an ankyrin repeat domain, wherein the recombinant binding protein is administered in 2 to 5 doses, with an interval of 25 to 35 days between each dose.
5. The method of item 4, wherein the disease of the retina is selected from exudative macular degeneration, polypoidal choroidal vasculopathy, retinal angiomatous proliferation, diabetic macular edema, pathological myopia, and branch retinal vein occlusion.
6. A method of reducing fluid in the retina, the method comprising the step of administering, to a patient in need of such reduction, about 0.25 mg to about 4 mg of a recombinant binding protein comprising an ankyrin repeat domain, wherein the recombinant binding protein is administered in 2 to 5 doses, with an interval of 25 to 35 days between each dose.
7. The method of item 6, wherein the fluid is selected from intrarentinal fluid, subretinal fluid, and fluid within intraretinal cysts.
8. A method of reducing retinal thickness, the method comprising the step of administering, to a patient in need of such reduction, about 0.25 mg to about 4 mg of a recombinant binding protein comprising an ankyrin repeat domain, wherein the recombinant binding protein is administered in 2 to 5 doses, with an interval of 25 to 35 days between each dose.
9. The method of any of items 1-8, wherein the binding protein is administered in 2 doses, with an interval of 25 to 35 days between each dose.
10. The method of any of items 1-8, wherein the binding protein is administered in 3 doses, with an interval of 25 to 35 days between each dose.
11. The method of any of items 1-8, wherein the binding protein is administered in 4 doses, with an interval of 25 to 35 days between each dose.
12. The method of any of items 1-8, wherein the binding protein is administered in 5 doses, with an interval of 25 to 35 days between each dose.
13. The method of any of items 1-8, wherein the method further comprises administering additional doses, following the 2 to 5 doses, with an interval of 25 to 115 days between each additional dose.
14. The method of item 13, wherein the binding protein is administered in 2 doses, with an interval of 25 to 35 days between each dose, followed by additional doses, with an interval of 50 to 115 days between each additional dose.
15. The method of item 14, wherein the binding protein is administered in 2 doses, with an interval of 25 to 35 days between each dose, followed by additional doses, with an interval of 50 to 90 days between each additional dose.
16. The method of item 14, wherein the binding protein is administered in 2 doses, with an interval of 25 to 35 days between each dose, followed by additional doses, with an interval of 50 to 60 days between each additional dose.
17. The method of item 14, wherein the binding protein is administered in 2 doses, with an interval of 25 to 35 days between each dose, followed by additional doses, with an interval of 80 to 90 days between each additional dose.
18. The method of item 14, wherein the binding protein is administered in 2 doses, with an interval of 25 to 35 days between each dose, followed by additional doses, with an interval of 105 to 115 days between each additional dose.
19. The method of item 13, wherein the binding protein is administered in 3 doses, with an interval of 25 to 35 days between each dose, followed by additional doses, with an interval of 50 to 115 days between each additional dose.
20. The method of item 19, wherein the binding protein is administered in 3 doses, with an interval of 25 to 35 days between each dose, followed by additional doses, with an interval of 50 to 90 days between each additional dose.
21. The method of item 19, wherein the binding protein is administered in 3 doses, with an interval of 25 to 35 days between each dose, followed by additional doses, with an interval of 50 to 60 days between each additional dose.
22. The method of item 19, wherein the binding protein is administered in 3 doses, with an interval of 25 to 35 days between each dose, followed by additional doses, with an interval of 80 to 90 days between each additional dose.
23. The method of item 19, wherein the binding protein is administered in 3 doses, with an interval of 25 to 35 days between each dose, followed by additional doses, with an interval of 105 to 115 days between each additional dose.
24. The method of item 13, wherein the binding protein is administered in 4 doses, with an interval of 25 to 35 days between each dose, followed by additional doses, with an interval of 50 to 115 days between each additional dose.
25. The method of item 24, wherein the binding protein is administered in 4 doses, with an interval of 25 to 35 days between each dose, followed by additional doses, with an interval of 50 to 90 days between each additional dose.
26. The method of item 24, wherein the binding protein is administered in 4 doses, with an interval of 25 to 35 days between each dose, followed by additional doses, with an interval of 50 to 60 days between each additional dose.
27. The method of item 24, wherein the binding protein is administered in 4 doses, with an interval of 25 to 35 days between each dose, followed by additional doses, with an interval of 80 to 90 days between each additional dose.
28. The method of item 24, wherein the binding protein is administered in 4 doses, with an interval of 25 to 35 days between each dose, followed by additional doses, with an interval of 105 to 115 days between each additional dose.
29. The method of item 13, wherein the binding protein is administered in 5 doses, with an interval of 25 to 35 days between each dose, followed by additional doses, with an interval of 50 to 115 days between each additional dose.
30. The method of item 29, wherein the binding protein is administered in 5 doses, with an interval of 25 to 35 days between each dose, followed by additional doses, with an interval of 50 to 90 days between each additional dose.
31. The method of item 29, wherein the binding protein is administered in 5 doses, with an interval of 25 to 35 days between each dose, followed by additional doses, with an interval of 50 to 60 days between each additional dose.
32. The method of item 29, wherein the binding protein is administered in 5 doses, with an interval of 25 to 35 days between each dose, followed by additional doses, with an interval of 80 to 90 days between each additional dose.
33. The method of item 29, wherein the binding protein is administered in 5 doses, with an interval of 25 to 35 days between each dose, followed by additional doses, with an interval of 105 to 115 days between each additional dose.
34. The method of any one of items 1 to 33, wherein the binding protein further comprises a polyethylene glycol moiety having a molecular weight of at least 5 kDa.
35. The method of item 34, wherein the polyethylene glycol moiety has a molecular weight selected from the group consisting of 5 kDA, 10 kDA, and 20 kDa.
36. The method of any one of items 1 to 35, wherein the N-terminal capping module of the ankyrin repeat domain comprises an Asp residue at position 5.
37. The method of any one of items 1 to 36, wherein the ankyrin repeat domain is selected from the group consisting of the ankyrin repeat domains of SEQ ID NO: 1, SEQ ID NO:2, SEQ ID NO:3, SEQ ID NO:4, SEQ ID NO:5, SEQ ID NO:6, SEQ ID NO:7.
38. The method of any one of items 1-37, wherein the binding protein comprises an ankyrin repeat domain comprising amino acids 1 to 126 of SEQ ID NO:3.
39. The method of any one of items 1-37, wherein the binding protein comprises an ankyrin repeat domain comprising a repeat module with an ankyrin repeat sequence motif selected from the group consisting of SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, and SEQ ID NO: 22.
40. The method of any one of items 1 to 39, wherein the binding domain binds VEGF-Axxx with a Kd below 10⁹M.
41. The method of any one of items 1 to 40, wherein the binding protein inhibits VEGF-Axxx binding to VEGFR-1.
42. The method of any one of items 1 to 41, wherein the ankyrin repeat domain is conjugated at its C-terminus via a peptide bond to a polypeptide linker and a C-terminal Cys residue, wherein the thiol of the C-terminal Cys is further conjugated to a maleimide-coupled polyethylene glycol.
43. The method of item 42, wherein the maleimide-coupled polyethylene glycol is α-[3-(3-maleimido-1-oxopropyl)amino]propyl-ω-methoxy-polyoxyethylene.
44. The method of item 42, wherein the polypeptide linker consists of 2 to 24 amino acids.
45. The method of any one of items 1 to 44, wherein the binding protein is administered with a pharmaceutically acceptable carrier and/or diluent.
46. The method of item 45, wherein the carrier is PBS.
47. The method of items 45 or 46, wherein the binding protein is administered by intravitreal injection.
48. The method of any one of items 1 to 47, wherein the binding protein is administered at a dose of about 0.25 mg, about 0.5 mg, about 1 mg, about 2 mg, about 3 mg, or about 4 mg.
49. The method of any one of items 1 and 9-48, wherein the method is effective in treating macular degeneration, polypoidal choroidal vasculopathy, retinal angiomatous proliferation, diabetic macular edema, pathological myopia, branch retinal vein occlusion, or central retinal vein occlusion in a patient having that condition.
50. The method of any one of items 1-49, wherein the patient's best corrected visual acuity improves by at least 15 letters after 12 weeks of beginning treatment.
51. The method of any one of items 1 to 50, wherein the patient experiences, after 4, 8, or 12 weeks of beginning treatment, a reduction of about 20%, about 25%, about 30%, about 35%, about 40%, about 45%, about 50%, about 55%, about 60%, about 65%, about 70%, about 75%, about 80%, about 85%, about 90%, about 95%, or about 100% of fluid in the central retina.
52. The method of any one of items 1 to 51, wherein the patient experiences, after 4, 8, or 12 weeks of beginning treatment, a reduction of about 20%, about 25%, about 30%, about 35%, about 40%, about 45%, about 50%, about 55%, about 60%, about 65%, about 70%, about 75%, about 80%, about 85%, about 90%, about 95%, or about 100% in one or more of intra retinal edema, intraretinal cysts, and sub-retinal fluid.
53. The method of any one of items 1 to 51, wherein the patient experiences, after 4, 8, or 12 weeks of beginning treatment, a reduction of at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, or at least about 95%, or at least about 99%, in one or more of intraretinal edema, intraretinal cysts, and sub-retinal fluid.
54. The method of item 52 or 53, wherein the reduction is assessed by spectral domain OCT.
55. The method of any one of items 1 to 54, wherein the patient experiences, after 4, 8, or 12 weeks of beginning treatment, a reduction in at least two, or in at least three, or in all of the following: central retina thickness, intraretinal edema, intraretinal cysts, or sub-retinal fluid.
56. The method of any one of items 1 to 55, wherein the patient is refractory to ranibizumab, bevacizumab, aflibercept, or pegaptanib therapy.
57. The method of item 56, wherein the patient has less than a 20% decrease in the center 1 mm² area of the macula after 3 intravitreal injections of ranibizumab, bevacizumab, or aflibercept.
58. The method of either item 56 or 57, wherein the patient is refractory to three, four, five, or six doses of ranizumab therapy.
59. The method of either item 56 or 57, wherein the patient is refractory to three, four, five, or six doses of bevacizumab therapy.
60. The method of either item 56 or 57, wherein the patient is refractory to three, four, five, or six doses of aflibercept therapy.
61. The method of either item 56 or 57, wherein the patient is refractory to three, four, five, or six doses of pegaptanib therapy.

SEQUENCE LISTING

<170>  PatentIn version 3.5

<210>  1
<211>  1025
<212>  PRT
<213>  Artificial

<220>
<223>  Ankyrin repeat protein

<400>  1

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Phe Asp Trp Met Gly Trp Thr Pro Leu His Leu Ala Ala His Glu Gly
        35                  40                  45

His Leu Glu Ile Val Glu Val Leu Leu Lys Asn Gly Ala Asp Val Asn
    50                  55                  60

Ala Thr Asp Val Ser Gly Tyr Thr Pro Leu His Leu Ala Ala Ala Asp
65                  70                  75                  80

Gly His Leu Glu Ile Val Glu Val Leu Leu Lys Tyr Gly Ala Asp Val
                85                  90                  95

Asn Thr Lys Asp Asn Thr Gly Trp Thr Pro Leu His Leu Ser Ala Asp
            100                 105                 110

Leu Gly Arg Leu Glu Ile Val Glu Val Leu Leu Lys Tyr Gly Ala Asp
        115                 120                 125

Val Asn Ala Gln Asp Lys Phe Gly Lys Thr Ala Phe Asp Ile Ser Ile
    130                 135                 140

Asp Asn Gly Asn Glu Asp Leu Ala Glu Ile Leu Gln Lys Ala Ala Ser
145                 150                 155                 160

Gly Ser Pro Ala Gly Ser Pro Thr Ser Thr Glu Glu Gly Thr Ser Glu
                165                 170                 175

Ser Ala Thr Pro Glu Ser Gly Pro Gly Thr Ser Thr Glu Pro Ser Glu
            180                 185                 190

Gly Ser Ala Pro Gly Ser Pro Ala Gly Ser Pro Thr Ser Thr Glu Glu
        195                 200                 205

Gly Thr Ser Thr Glu Pro Ser Glu Gly Ser Ala Pro Gly Thr Ser Thr
    210                 215                 220

Glu Pro Ser Glu Gly Ser Ala Pro Gly Thr Ser Glu Ser Ala Thr Pro
225                 230                 235                 240

Glu Ser Gly Pro Gly Ser Glu Pro Ala Thr Ser Gly Ser Glu Thr Pro
                245                 250                 255

Gly Ser Glu Pro Ala Thr Ser Gly Ser Glu Thr Pro Gly Ser Pro Ala
            260                 265                 270

Gly Ser Pro Thr Ser Thr Glu Glu Gly Thr Ser Glu Ser Ala Thr Pro
        275                 280                 285

Glu Ser Gly Pro Gly Thr Ser Thr Glu Pro Ser Glu Gly Ser Ala Pro
    290                 295                 300

Gly Thr Ser Thr Glu Pro Ser Glu Gly Ser Ala Pro Gly Ser Pro Ala
305                 310                 315                 320

Gly Ser Pro Thr Ser Thr Glu Glu Gly Thr Ser Thr Glu Pro Ser Glu
                325                 330                 335

Gly Ser Ala Pro Gly Thr Ser Thr Glu Pro Ser Glu Gly Ser Ala Pro
            340                 345                 350

Gly Thr Ser Glu Ser Ala Thr Pro Glu Ser Gly Pro Gly Thr Ser Thr
        355                 360                 365

Glu Pro Ser Glu Gly Ser Ala Pro Gly Thr Ser Glu Ser Ala Thr Pro
    370                 375                 380

Glu Ser Gly Pro Gly Ser Glu Pro Ala Thr Ser Gly Ser Glu Thr Pro
385                 390                 395                 400

Gly Thr Ser Thr Glu Pro Ser Glu Gly Ser Ala Pro Gly Thr Ser Thr
                405                 410                 415

Glu Pro Ser Glu Gly Ser Ala Pro Gly Thr Ser Glu Ser Ala Thr Pro
            420                 425                 430

Glu Ser Gly Pro Gly Thr Ser Glu Ser Ala Thr Pro Glu Ser Gly Pro
        435                 440                 445

Gly Ser Pro Ala Gly Ser Pro Thr Ser Thr Glu Glu Gly Thr Ser Glu
    450                 455                 460

Ser Ala Thr Pro Glu Ser Gly Pro Gly Ser Glu Pro Ala Thr Ser Gly
465                 470                 475                 480

Ser Glu Thr Pro Gly Thr Ser Glu Ser Ala Thr Pro Glu Ser Gly Pro
                485                 490                 495

Gly Thr Ser Thr Glu Pro Ser Glu Gly Ser Ala Pro Gly Thr Ser Thr
            500                 505                 510

Glu Pro Ser Glu Gly Ser Ala Pro Gly Thr Ser Thr Glu Pro Ser Glu
        515                 520                 525

Gly Ser Ala Pro Gly Thr Ser Thr Glu Pro Ser Glu Gly Ser Ala Pro
    530                 535                 540

Gly Thr Ser Thr Glu Pro Ser Glu Gly Ser Ala Pro Gly Thr Ser Thr
545                 550                 555                 560

Glu Pro Ser Glu Gly Ser Ala Pro Gly Ser Pro Ala Gly Ser Pro Thr
                565                 570                 575

Ser Thr Glu Glu Gly Thr Ser Thr Glu Pro Ser Glu Gly Ser Ala Pro
            580                 585                 590

Gly Thr Ser Glu Ser Ala Thr Pro Glu Ser Gly Pro Gly Ser Glu Pro
        595                 600                 605

Ala Thr Ser Gly Ser Glu Thr Pro Gly Thr Ser Glu Ser Ala Thr Pro
    610                 615                 620

Glu Ser Gly Pro Gly Ser Glu Pro Ala Thr Ser Gly Ser Glu Thr Pro
625                 630                 635                 640

Gly Thr Ser Glu Ser Ala Thr Pro Glu Ser Gly Pro Gly Thr Ser Thr
                645                 650                 655

Glu Pro Ser Glu Gly Ser Ala Pro Gly Thr Ser Glu Ser Ala Thr Pro
            660                 665                 670

Glu Ser Gly Pro Gly Ser Pro Ala Gly Ser Pro Thr Ser Thr Glu Glu
        675                 680                 685

Gly Ser Pro Ala Gly Ser Pro Thr Ser Thr Glu Glu Gly Ser Pro Ala
    690                 695                 700

Gly Ser Pro Thr Ser Thr Glu Glu Gly Thr Ser Glu Ser Ala Thr Pro
705                 710                 715                 720

Glu Ser Gly Pro Gly Thr Ser Thr Glu Pro Ser Glu Gly Ser Ala Pro
                725                 730                 735

Gly Thr Ser Glu Ser Ala Thr Pro Glu Ser Gly Pro Gly Ser Glu Pro
            740                 745                 750

Ala Thr Ser Gly Ser Glu Thr Pro Gly Thr Ser Glu Ser Ala Thr Pro
        755                 760                 765

Glu Ser Gly Pro Gly Ser Glu Pro Ala Thr Ser Gly Ser Glu Thr Pro
    770                 775                 780

Gly Thr Ser Glu Ser Ala Thr Pro Glu Ser Gly Pro Gly Thr Ser Thr
785                 790                 795                 800

Glu Pro Ser Glu Gly Ser Ala Pro Gly Ser Pro Ala Gly Ser Pro Thr
                805                 810                 815

Ser Thr Glu Glu Gly Thr Ser Glu Ser Ala Thr Pro Glu Ser Gly Pro
            820                 825                 830

Gly Ser Glu Pro Ala Thr Ser Gly Ser Glu Thr Pro Gly Thr Ser Glu
        835                 840                 845

Ser Ala Thr Pro Glu Ser Gly Pro Gly Ser Pro Ala Gly Ser Pro Thr
    850                 855                 860

Ser Thr Glu Glu Gly Ser Pro Ala Gly Ser Pro Thr Ser Thr Glu Glu
865                 870                 875                 880

Gly Thr Ser Thr Glu Pro Ser Glu Gly Ser Ala Pro Gly Thr Ser Glu
                885                 890                 895

Ser Ala Thr Pro Glu Ser Gly Pro Gly Thr Ser Glu Ser Ala Thr Pro
            900                 905                 910

Glu Ser Gly Pro Gly Thr Ser Glu Ser Ala Thr Pro Glu Ser Gly Pro
        915                 920                 925

Gly Ser Glu Pro Ala Thr Ser Gly Ser Glu Thr Pro Gly Ser Glu Pro
    930                 935                 940

Ala Thr Ser Gly Ser Glu Thr Pro Gly Ser Pro Ala Gly Ser Pro Thr

945                 950                 955                 960

Ser Thr Glu Glu Gly Thr Ser Thr Glu Pro Ser Glu Gly Ser Ala Pro
                965                 970                 975

Gly Thr Ser Thr Glu Pro Ser Glu Gly Ser Ala Pro Gly Ser Glu Pro
            980                 985                 990

Ala Thr Ser Gly Ser Glu Thr Pro  Gly Thr Ser Glu Ser  Ala Thr Pro
        995                 1000                1005

Glu Ser  Gly Pro Gly Thr Ser  Thr Glu Pro Ser Glu  Gly Ser Ala
    1010                1015                1020

Pro Gly
    1025

<210>  2
<211>  168
<212>  PRT
<213>  Artificial

<220>
<223>  Ankyrin repeat protein

<400>  2

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Phe Asp Trp Met Gly Trp Thr Pro Leu His Leu Ala Ala His Glu Gly
        35                  40                  45

His Leu Glu Ile Val Glu Val Leu Leu Lys Asn Gly Ala Asp Val Asn
    50                  55                  60

Ala Thr Asp Val Ser Gly Tyr Thr Pro Leu His Leu Ala Ala Ala Asp
65                  70                  75                  80

Gly His Leu Glu Ile Val Glu Val Leu Leu Lys His Gly Ala Asp Val
                85                  90                  95

Asn Thr Lys Asp Asn Thr Gly Trp Thr Pro Leu His Leu Ser Ala Asp
            100                 105                 110

Leu Gly His Leu Glu Ile Val Glu Val Leu Leu Lys Asn Gly Ala Asp
        115                 120                 125

Val Asn Ala Gln Asp Lys Phe Gly Lys Thr Ala Phe Asp Ile Ser Ile
    130                 135                 140

Asp Asn Gly Asn Glu Asp Leu Ala Glu Ile Leu Gln Lys Ala Ala Gly
145                 150                 155                 160

Gly Gly Ser Gly Gly Gly Ser Cys
                165

<210>  3
<211>  135
<212>  PRT
<213>  Artificial

<220>
<223>  Ankyrin repeat protein

<400>  3

Gly Ser Asp Leu Asp Lys Lys Leu Leu Glu Ala Ala Arg Ala Gly Gln
1               5                   10                  15

Asp Asp Glu Val Arg Ile Leu Met Ala Asn Gly Ala Asp Val Asn Ala
            20                  25                  30

Arg Asp Ser Thr Gly Trp Thr Pro Leu His Leu Ala Ala Pro Trp Gly
        35                  40                  45

His Pro Glu Ile Val Glu Val Leu Leu Lys Asn Gly Ala Asp Val Asn
    50                  55                  60

Ala Ala Asp Phe Gln Gly Trp Thr Pro Leu His Leu Ala Ala Ala Val
65                  70                  75                  80

Gly His Leu Glu Ile Val Glu Val Leu Leu Lys Tyr Gly Ala Asp Val
                85                  90                  95

Asn Ala Gln Asp Lys Phe Gly Lys Thr Ala Phe Asp Ile Ser Ile Asp
            100                 105                 110

Asn Gly Asn Glu Asp Leu Ala Glu Ile Leu Gln Lys Ala Ala Gly Gly
        115                 120                 125

Gly Ser Gly Gly Gly Ser Cys
    130                 135

<210>  4
<211>  540
<212>  PRT

<213>  Artificial

<220>
<223>  Ankyrin repeat protein

<400>  4

Gly Ser Asp Leu Gly Lys Lys Leu Leu Glu Ala Ala Arg Ala Gly Gln
1               5                   10                  15

Asp Asp Glu Val Arg Ile Leu Met Ala Asn Gly Ala Asp Val Asn Thr
            20                  25                  30

Ala Asp Ser Thr Gly Trp Thr Pro Leu His Leu Ala Val Pro Trp Gly
        35                  40                  45

His Leu Glu Ile Val Glu Val Leu Leu Lys Tyr Gly Ala Asp Val Asn
    50                  55                  60

Ala Lys Asp Phe Gln Gly Trp Thr Pro Leu His Leu Ala Ala Ala Ile
65                  70                  75                  80

Gly His Gln Glu Ile Val Glu Val Leu Leu Lys Asn Gly Ala Asp Val
                85                  90                  95

Asn Ala Gln Asp Lys Phe Gly Lys Thr Ala Phe Asp Ile Ser Ile Asp
            100                 105                 110

Asn Gly Asn Glu Asp Leu Ala Glu Ile Leu Gln Lys Ala Ala Gly Ser
        115                 120                 125

Gly Ser Ala Ser Pro Ala Ala Pro Ala Pro Ala Ser Pro Ala Ala Pro
    130                 135                 140

Ala Pro Ser Ala Pro Ala Ala Ser Pro Ala Ala Pro Ala Pro Ala Ser
145                 150                 155                 160

Pro Ala Ala Pro Ala Pro Ser Ala Pro Ala Ala Ser Pro Ala Ala Pro
                165                 170                 175

Ala Pro Ala Ser Pro Ala Ala Pro Ala Pro Ser Ala Pro Ala Ala Ser
            180                 185                 190

Pro Ala Ala Pro Ala Pro Ala Ser Pro Ala Ala Pro Ala Pro Ser Ala
        195                 200                 205

Pro Ala Ala Ser Pro Ala Ala Pro Ala Pro Ala Ser Pro Ala Ala Pro
    210                 215                 220

Ala Pro Ser Ala Pro Ala Ala Ser Pro Ala Ala Pro Ala Pro Ala Ser
225                 230                 235                 240

Pro Ala Ala Pro Ala Pro Ser Ala Pro Ala Ala Ser Pro Ala Ala Pro
                245                 250                 255

Ala Pro Ala Ser Pro Ala Ala Pro Ala Pro Ser Ala Pro Ala Ala Ser
            260                 265                 270

Pro Ala Ala Pro Ala Pro Ala Ser Pro Ala Ala Pro Ala Pro Ser Ala
        275                 280                 285

Pro Ala Ala Ser Pro Ala Ala Pro Ala Pro Ala Ser Pro Ala Ala Pro
    290                 295                 300

Ala Pro Ser Ala Pro Ala Ala Ser Pro Ala Ala Pro Ala Pro Ala Ser
305                 310                 315                 320

Pro Ala Ala Pro Ala Pro Ser Ala Pro Ala Ala Ser Pro Ala Ala Pro
                325                 330                 335

Ala Pro Ala Ser Pro Ala Ala Pro Ala Pro Ser Ala Pro Ala Ala Ser
            340                 345                 350

Pro Ala Ala Pro Ala Pro Ala Ser Pro Ala Ala Pro Ala Pro Ser Ala
        355                 360                 365

Pro Ala Ala Ser Pro Ala Ala Pro Ala Pro Ala Ser Pro Ala Ala Pro
    370                 375                 380

Ala Pro Ser Ala Pro Ala Ala Ser Pro Ala Ala Pro Ala Pro Ala Ser
385                 390                 395                 400

Pro Ala Ala Pro Ala Pro Ser Ala Pro Ala Ala Ser Pro Ala Ala Pro
                405                 410                 415

Ala Pro Ala Ser Pro Ala Ala Pro Ala Pro Ser Ala Pro Ala Ala Ser
            420                 425                 430

Pro Ala Ala Pro Ala Pro Ala Ser Pro Ala Ala Pro Ala Pro Ser Ala
        435                 440                 445

Pro Ala Ala Ser Pro Ala Ala Pro Ala Pro Ala Ser Pro Ala Ala Pro
    450                 455                 460

Ala Pro Ser Ala Pro Ala Ala Ser Pro Ala Ala Pro Ala Pro Ala Ser
465                 470                 475                 480

Pro Ala Ala Pro Ala Pro Ser Ala Pro Ala Ala Ser Pro Ala Ala Pro
                485                 490                 495

Ala Pro Ala Ser Pro Ala Ala Pro Ala Pro Ser Ala Pro Ala Ala Ser
            500                 505                 510

Pro Ala Ala Pro Ala Pro Ala Ser Pro Ala Ala Pro Ala Pro Ser Ala
        515                 520                 525

Pro Ala Ala Ser Pro Ala Ala Pro Ala Pro Ala Ser
    530                 535                 540

<210>  5
<211>  135
<212>  PRT
<213>  Artificial

<220>
<223>  Ankyrin repeat protein

<400>  5

Gly Ser Asp Leu Gly Lys Lys Leu Leu Glu Ala Ala Arg Val Gly Gln
1               5                   10                  15

Asp Asp Glu Val Arg Ile Leu Met Ala Asp Gly Ala Asp Val Asn Ala
            20                  25                  30

Ser Asp Phe Lys Gly Asp Thr Pro Leu His Leu Ala Ala Ser Gln Gly
        35                  40                  45

His Leu Glu Ile Val Glu Val Leu Leu Lys Tyr Gly Ala Asp Val Asn
    50                  55                  60

Ala Tyr Asp Met Leu Gly Trp Thr Pro Leu His Leu Ala Ala Asp Leu
65                  70                  75                  80

Gly His Leu Glu Ile Val Glu Val Leu Leu Lys Tyr Gly Ala Asp Val
                85                  90                  95

Asn Ala Gln Asp Arg Phe Gly Lys Thr Ala Phe Asp Ile Ser Ile Asp
            100                 105                 110

Asn Gly Asn Glu Asp Leu Ala Glu Ile Leu Gln Lys Ala Ala Gly Ser
        115                 120                 125

Pro Ser Thr Ala Asp Gly Cys
    130                 135

<210>  6
<211>  130
<212>  PRT
<213>  Artificial

<220>
<223>  Ankyrin repeat protein

<400>  6

Gly Ser Asp Leu Gly Lys Lys Leu Leu Glu Ala Val Arg Ala Gly Gln
1               5                   10                  15

Asp Asp Glu Val Arg Ile Leu Met Thr Asn Gly Ala Asp Val Asn Ala
            20                  25                  30

Lys Asp Gln Phe Gly Phe Thr Pro Leu Gln Leu Ala Ala Tyr Asn Gly
        35                  40                  45

His Leu Glu Ile Val Glu Val Leu Leu Lys Tyr Gly Ala Asp Val Asn
    50                  55                  60

Ala Phe Asp Ile Phe Gly Trp Thr Pro Leu His Leu Ala Ala Asp Leu
65                  70                  75                  80

Gly His Leu Glu Ile Val Glu Val Leu Leu Lys Asn Gly Ala Asp Val
                85                  90                  95

Asn Ala Gln Asp Lys Phe Gly Arg Thr Ala Phe Asp Ile Ser Ile Asp
            100                 105                 110

Asn Gly Asn Glu Asp Leu Ala Glu Ile Leu Gln Lys Ala Ala Ser Gly
        115                 120                 125

Ser Cys
    130

<210>  7
<211>  168
<212>  PRT
<213>  Artificial

<220>
<223>  Ankyrin repeat protein

<400>  7
   Gly Ser Asp Leu Gly Lys Lys Leu Leu Glu Ala Ala Arg Ala Gly Gln
   1               5                   10                  15
   Asp Asp Glu Val Arg Ile Leu Met Ala Asn Gly Ala Asp Val Asn Ala
               20                  25                  30
   Val Asp Tyr Ile Gly Trp Thr Pro Leu His Leu Ala Ala Ala Tyr Gly
           35                  40                  45
   His Leu Glu Ile Val Glu Val Leu Leu Lys Tyr Ser Ala Asp Val Asn
       50                  55                  60
   Ala Glu Asp Phe Ala Gly Tyr Thr Pro Leu His Leu Ala Ala Ser Asn
   65                  70                  75                  80
   Gly His Leu Glu Ile Val Glu Val Leu Leu Lys Tyr Gly Ala Asp Val
                   85                  90                  95
   Asn Thr Lys Asp Asn Thr Gly Trp Thr Pro Leu His Leu Ser Ala Asp
               100                 105                 110
   Leu Gly His Leu Glu Ile Val Glu Val Leu Leu Lys Tyr Gly Ala Asp
           115                 120                 125
   Val Asn Thr Gln Asp Lys Phe Gly Lys Thr Ala Phe Asp Ile Ser Ile
       130                 135                 140
   Asp Asn Gly Asn Glu Asp Leu Ala Glu Ile Leu Gln Lys Ala Ala Gly
   145                 150                 155                 160
   Ser Pro Ser Thr Ala Asp Gly Cys
                   165

<210>  8
<211>  9
<212>  PRT
<213>  Artificial

<220>
<223>  Polypeptide linker

<400>  8

Gly Gly Gly Ser Gly Gly Gly Ser Cys
1               5

<210>  9
<211>  9
<212>  PRT
<213>  Artificial

<220>
<223>  Polypeptide linker

<400>  9

Gly Ser Pro Ser Thr Ala Asp Gly Cys
1               5

<210>  10
<211>  33
<212>  PRT
<213>  Artificial

<220>
<223>  Ankyrin repeat sequence motif


<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (27)..(27)
<223>  Xaa can be any naturally occurring amino acid

<400>  10

Xaa Asp Xaa Xaa Gly Xaa Thr Pro Leu His Leu Ala Ala Xaa Xaa Gly
1               5                   10                  15


His Leu Glu Ile Val Glu Val Leu Leu Lys Xaa Gly Ala Asp Val Asn
            20                  25                  30


Ala

<210>  11
<211>  33
<212>  PRT
<213>  Artificial

<220>
<223>  Ankyrin repeat sequence motif


<220>
<221>  misc_feature
<222>  (1)..(1)
<223>  Xaa can be any naturally occurring amino acid

<220>
<221>  misc_feature

<222>  (3)..(4)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (27)..(27)
<223>  Xaa can be any naturally occurring amino acid

<400>  11

Xaa Asp Xaa Xaa Gly Trp Thr Pro Leu His Leu Ala Ala Xaa Xaa Gly
1               5                   10                  15

His Leu Glu Ile Val Glu Val Leu Leu Lys Xaa Gly Ala Asp Val Asn
            20                  25                  30

Ala

<210>  12
<211>  33
<212>  PRT
<213>  Artificial

<220>
<223>  Ankyrin repeat sequence motif

<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (27)..(27)
<223>  Xaa can be any naturally occurring amino acid

<220>
<221>  misc_feature
<222>  (33)..(33)
<223>  Xaa can be any naturally occurring amino acid

<400>  12

Xaa Asp Xaa Xaa Gly Xaa Thr Pro Leu His Leu Ala Ala Xaa Xaa Gly
1               5                   10                  15

His Leu Glu Ile Val Glu Val Leu Leu Lys Xaa Gly Ala Asp Val Asn
            20                  25                  30

Xaa

<210>  13
<211>  33
<212>  PRT
<213>  Artificial

<220>
<223>  Ankyrin repeat sequence motif

<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (12)..(12)
<223>  Xaa can be any naturally occurring amino acid

<220>
<221>  misc_feature
<222>  (17)..(17)
<223>  Xaa can be any naturally occurring amino acid

<220>
<221>  misc_feature
<222>  (27)..(27)
<223>  Xaa can be any naturally occurring amino acid

<220>
<221>  misc_feature
<222>  (33)..(33)
<223>  Xaa can be any naturally occurring amino acid

<400>  13

Xaa Asp Xaa Xaa Gly Trp Thr Pro Leu His Leu Xaa Ala Asp Leu Gly
1               5                   10                  15

Xaa Leu Glu Ile Val Glu Val Leu Leu Lys Xaa Gly Ala Asp Val Asn
            20                  25                  30

Xaa

<210>  14
<211>  33
<212>  PRT
<213>  Artificial

<220>
<223>  Ankyrin repeat sequence motif



<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (18)..(18)
<223>  Xaa can be any naturally occurring amino acid

<220>
<221>  misc_feature
<222>  (27)..(27)
<223>  Xaa can be any naturally occurring amino acid

<400>  14

Xaa Asp Xaa Xaa Gly Xaa Thr Pro Leu His Leu Ala Ala Xaa Xaa Gly
1               5                   10                  15


His Xaa Glu Ile Val Glu Val Leu Leu Lys Xaa Gly Ala Asp Val Asn
            20                  25                  30


Ala

<210>  15
<211>  33
<212>  PRT
<213>  Artificial

<220>
<223>  Ankyrin repeat sequence motif



<220>

<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (27)..(27)
<223>  Xaa can be any naturally occurring amino acid

<400>  15

Xaa Asp Xaa Xaa Gly Xaa Thr Pro Leu His Leu Ala Ala Xaa Xaa Gly
1               5                   10                  15

His Leu Glu Ile Val Glu Val Leu Leu Lys Xaa Gly Ala Asp Val Asn
            20                  25                  30

Ala

<210>  16
<211>  33
<212>  PRT
<213>  Artificial

<220>
<223>  Ankyrin repeat sequence motif

<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (27)..(27)

<223>  Xaa can be any naturally occurring amino acid

<400>  16

Xaa Asp Xaa Xaa Gly Trp Thr Pro Leu His Leu Ala Ala Xaa Xaa Gly
1               5                   10                  15

His Leu Glu Ile Val Glu Val Leu Leu Lys Xaa Gly Ala Asp Val Asn
            20                  25                  30

Ala

<210>  17
<211>  33
<212>  PRT
<213>  Artificial

<220>
<223>  Ankyrin repeat sequence motif

<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (27)..(27)
<223>  Xaa can be any naturally occurring amino acid

<220>
<221>  misc_feature
<222>  (33)..(33)
<223>  Xaa can be any naturally occurring amino acid

<400>  17

Xaa Asp Xaa Xaa Gly Xaa Thr Pro Leu His Leu Ala Ala Xaa Xaa Gly
1               5                   10                  15

His Leu Glu Ile Val Glu Val Leu Leu Lys Xaa Gly Ala Asp Val Asn
            20                  25                  30

Xaa

<210> 18
<211> 33
<212> PRT
<213> Artificial

<220>
<223> Ankyrin repeat sequence motif


<220>
<221> misc_feature
<222> (1)..(1)
<223> Xaa can be any naturally occurring amino acid

<220>
<221> misc_feature
<222> (3)..(4)
<223> Xaa can be any naturally occurring amino acid

<220>
<221> misc_feature
<222> (6)..(6)
<223> Xaa can be any naturally occurring amino acid

<220>
<221> misc_feature
<222> (12)..(12)
<223> Xaa can be any naturally occurring amino acid

<220>
<221> misc_feature
<222> (14)..(15)
<223> Xaa can be any naturally occurring amino acid

<220>
<221> misc_feature
<222> (27)..(27)
<223> Xaa can be any naturally occurring amino acid

<400> 18

Xaa Asp Xaa Xaa Gly Xaa Thr Pro Leu His Leu Xaa Ala Xaa Xaa Gly
1               5                   10                  15


His Leu Glu Ile Val Glu Val Leu Leu Lys Xaa Gly Ala Asp Val Asn
            20                  25                  30


Ala

<210> 19
<211> 33
<212> PRT
<213> Artificial

<220>
<223> Ankyrin repeat sequence motif


<220>

<221>  misc_feature
<222>  (1)..(1)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (18)..(18)
<223>  Xaa can be any naturally occurring amino acid

<220>
<221>  misc_feature
<222>  (27)..(27)
<223>  Xaa can be any naturally occurring amino acid

<400>  19

Xaa Asp Phe Lys Xaa Asp Thr Pro Leu His Leu Ala Ala Xaa Xaa Gly
1               5                   10                  15

His Xaa Glu Ile Val Glu Val Leu Leu Lys Xaa Gly Ala Asp Val Asn
            20                  25                  30

Ala

<210>  20
<211>  33
<212>  PRT
<213>  Artificial

<220>
<223>  Ankyrin repeat sequence motif

<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (13)..(15)

<223>  Xaa can be any naturally occurring amino acid

<220>
<221>  misc_feature
<222>  (27)..(27)
<223>  Xaa can be any naturally occurring amino acid

<400>  20

Xaa Asp Xaa Leu Xaa Xaa Thr Pro Leu His Leu Ala Xaa Xaa Xaa Gly
1               5                   10                  15


His Leu Glu Ile Val Glu Val Leu Leu Lys Xaa Gly Ala Asp Val Asn
            20                  25                  30


Ala

<210>  21
<211>  33
<212>  PRT
<213>  Artificial

<220>
<223>  Ankyrin repeat sequence motif



<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (14)..(15)
<223>  Xaa can be any naturally occurring amino acid

<220>
<221>  misc_feature
<222>  (27)..(27)
<223>  Xaa can be any naturally occurring amino acid

<400>  21

Xaa Asp Xaa Xaa Gly Xaa Thr Pro Leu Xaa Leu Ala Ala Xaa Xaa Gly
1               5                   10                  15

His Leu Glu Ile Val Glu Val Leu Leu Lys Xaa Gly Ala Asp Val Asn
            20                  25                  30

Ala

<210>  22
<211>  33
<212>  PRT
<213>  Artificial

<220>
<223>  Ankyrin repeat sequence motif

<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (17)..(17)
<223>  Xaa can be any naturally occurring amino acid

<220>
<221>  misc_feature
<222>  (27)..(27)
<223>  Xaa can be any naturally occurring amino acid

<400>  22

Xaa Asp Xaa Xaa Gly Trp Thr Xaa Leu His Leu Ala Ala Asp Leu Gly
1               5                   10                  15

Xaa Leu Glu Ile Val Glu Val Leu Leu Lys Xaa Gly Ala Asp Val Asn
            20                  25                  30

Ala

## Claims

1. A recombinant binding protein comprising an ankyrin repeat domain, wherein the ankyrin domain binds VEGF-Axxx with a Kd below 10⁷ M, for use in a method of inhibiting binding between VEGF-Axxx and VEGFR-2, wherein said recombinant binding protein is to be administered to a patient in need of such inhibition at 50 - 115 day intervals, at a dose of 1 mg to 4 mg, wherein the use is for treating age-related macular degeneration, diabetic macular edema, pathological myopia, branch retinal vein occlusion, or central retinal vein occlusion in a patient having that condition.

2. The recombinant binding protein for use of claim 1, wherein the binding protein further comprises a polyethylene glycol moiety of at least 5 kDa molecular weight, preferably wherein the N-terminal capping module of the ankyrin repeat domain comprises an Asp residue at position 5.

3. The recombinant binding protein for use of any of claims 1 or 2, wherein the ankyrin repeat domain competes for binding to VEGF-Axxx with the ankyrin repeat domains of SEQ ID NO:1 or 3.

4. The recombinant binding protein for use of any of claims 1 or 2, wherein the ankyrin repeat domain is selected from the group consisting of the ankyrin repeat domains of SEQ ID NOS: 1 to 7.

5. The recombinant binding protein for use of any one of claims 1 to 4, wherein the binding protein inhibits VEGF-Axxx binding to VEGFR-1.

6. The recombinant binding protein for use of any one of claims 1 to 5, wherein the ankyrin repeat domain is conjugated at its C-terminus via a peptide bond to a polypeptide linker and a C-terminal Cys residue, wherein the thiol of the C-terminal Cys is further conjugated to a maleimide-coupled polyethylene glycol and wherein the maleimide-coupled polyethylene glycol is preferably α-[3-(3-maleimido-1-oxopropyl)amino]propyl-ω-methoxy-polyoxyethylene.

7. The recombinant binding protein for use of any one of claims 1-6, wherein the binding protein is an ankyrin repeat protein selected from the group consisting of the ankyrin repeat proteins of SEQ ID NOS: 2, 3, 5, 6 or 7.

8. The recombinant binding protein for use of claim 6, wherein the polyethylene glycol moiety has a molecular weight of 20 kDa.

9. The recombinant binding protein for use of claim 1, wherein the binding protein comprises the ankyrin repeat protein of SEQ ID NO:3 wherein the thiol of the C-terminal Cys of the ankyrin repeat protein is further conjugated to a maleimide-coupled polyethylene glycol.

10. The recombinant binding protein for use of claim 9, wherein the maleimide-coupled polyethylene glycol is α-[3-(3-maleimido-1-oxopropyl)amino]propyl-ω-methoxy-polyoxyethylene, and wherein the polyethylene glycol moiety has a molecular weight of at least 10 kDa.

11. The recombinant binding protein for use of any one of claims 1 to 10, wherein the binding protein is administered with a pharmaceutically acceptable carrier and/or diluent, wherein the carrier is preferably PBS.

12. The recombinant binding protein for use of claim 11, wherein the binding protein is to be administered by intravitreal injection.
